# EUROPEAN PATENT APPLICATION

(11) **EP 3 854 871 A1**
(43) Date of publication of application: **28.07.2021**
(21) Application number: 19863140.0
(22) Date of filing: 23.09.2019
(51) Int. Cl.: C12N 5/10, C12N 9/22, C12N 15/87

(54) **METHOD FOR GENE EDITING OF CELL ON THE BASIS OF CRISPR/CAS SYSTEM**

(30) Priority: 21.09.2018 CN 201811117875; 28.09.2018 CN 201811140870; 29.08.2019 CN 201910809475; 29.08.2019 CN 201910809598
(71) Applicant: CAFA THERAPEUTICS LIMITED, Dublin D01 AE27 (IE)
(72) Inventor: LI, Zonghai, Shanghai 200231 (CN); LIAO, Zhaohui, Shanghai 200231 (CN)
(74) Representative: Lavoix
(86) International application number: PCT/CN2019/107374
(87) International publication number: WO 2020/057668

(57) **Abstract**

Provided is a method for gene editing of a cell on the basis of a CRISPR/Cas system. The Cas enzyme is a Cas9 enzyme having an enzyme activity of 0.1-1 nmol. Further provided are a method for constructing a universal T cell, a T cell so prepared and use thereof. TCR genes and MHC genes of a T cell are edited by means of gene editing technology. Further provided is a gRNA construct.

## Description

### FIELD OF THE INVENTION

The present invention relates to a method of gene editing. More specifically, it relates to a method for gene editing of cells using the CRISPR/Cas system.

### BACKGROUND OF THE INVENTION

Gene editing includes changing the genome by deleting, inserting, and mutating or replacing specific nucleic acid sequences. The CRISPR-Cas system consists of Clustered Regularly Interspaced Short Palindromic Repeats (CRISPR) and the associated Cas protein. RNA-guided Cas endonuclease specifically targets and cleaves DNA in a sequence-dependent manner (Jinek, M. et al., "A programmable dual-RNA-guided DNA endonuclease in adaptive bacterial immunity," Science 337, 816- 821 (2012); Sternberg, S. H. et al., "DNA interrogation by the CRISPR RNA-guided endonuclease Cas9," Nature 507, 62 (2014)), and has been widely used in gene editing in various organisms and model systems.

However, there is still a problem of low gene editing efficiency in the process of gene editing. For example, when CRISPR-Cas9 edits T cells, as T cells are terminally differentiated primary cells and the time window for *in vitro* amplification is limited, the gene transfecting efficiency is relatively low, such as that disclosed in Clin Cancer Res; 23(9) May 1, 2017, wherein the efficiency of knocking out the coding gene of TCR receptor or HLA protein alone can reach about 80%, while the efficiency of simultaneous knockout of both is merely about 60%.

Therefore, how to knock out quickly and efficiently, or knock out multiple genes quickly and efficiently at one time in cells, has become a difficult point in this field.

### SUMMARY OF THE INVENTION

The purpose of the present invention is to provide a rapid and efficient method to knock out genes in cells, especially a rapid and efficient method to knock out multiple genes at once.

In the first aspect of the present invention, provided is a method for gene editing of cells based on a CRISPR/Cas system. A complex of a Cas enzyme and a gRNA is introduced into the cell for gene editing, wherein the ratio of the Cas enzyme to the gRNA in the complex is 1:3∼1:5.

In a specific embodiment, the Cas enzyme is a Cas9 enzyme.

In a specific embodiment, enzyme activity of the Cas9 enzyme is 0.1 to 1 nmol, preferably 0.2 to 0.7 nmol, more preferably 0.3 to 0.5 nmol, and most preferably 0.37 nmol.

In a specific embodiment, the Cas enzyme is the Cas9 enzyme, and in the complex, the molar ratio of the Cas9 enzyme to the gRNA is 1:1 to 1:10, preferably 1:3 to 1:5, and more preferably 1:4.

In the present invention, for example, the Cas9 enzyme from NEB Company can be used. Of course, those skilled in the art can select other Cas9 enzymes with the same or similar functions.

In a specific embodiment, the function that the Cas9 enzyme can achieve is that, in 30 µl of a reacted Cas9 enzyme reaction system (the reaction system including: 20 mM HEPES, 100 mM NaCl, 5 mM MgCl₂, 0.1 mM EDTA and at 25°C, pH is 6.5), under the condition that 1 nM PvuII linearized pBR322 DNA (with a target site CGCTTGTTTCGGCGTGGGTA), 40 nM sgRNA and 20 nM of the Cas9 enzyme are contained, in the case of incubation at 37°C for 1 hour, 90% of pBR322 DNA is comfirmed to be degraded through agarose gel electrophoresis. In this reaction system, the amount of the Cas9 enzyme that catalyzes the complete conversion of 1 nmol substrate (pBR322 DNA linearized by PvuII) into products in 1 minute is 0.37 nmol, and the amount of the Cas9 enzyme is 59.57ng. The enzyme activity of the Cas9 enzyme is 0.37 nmol (the amount of enzyme that catalyzes the conversion of 1 nmol of substrate into products in 1 minute). In the present invention, if the enzyme of NEB is taken as an example, the enzyme activity of the enzyme is 0.37 nmol.

Those skilled in the art can understand that the calculation of the molar ratio of the Cas9 enzyme and the gRNA desired to be introduced and the determination of the concentration of the Cas9 enzyme in the introduced complex are based on the above Cas9 enzyme activity herein. When the activity of the Cas9 enzyme changes, those skilled in the art can select the concentration of the Cas9 enzyme and its molar ratio to the gRNA by conversion based on the ratio determined herein and according to the description of the activity in specifications of different enzymes.

Those skilled in the art can also understand that the above-mentioned Cas enzyme with an enzyme activity of 0.37 nmol is only an example. For other Cas9 enzymes, if the enzyme activity is different from the Cas enzyme, those skilled in the art can calculate according to the enzyme activity to confirm the amount of the Cas9 enzyme to be used and its molar ratio to the gRNA.

In a specific embodiment, the present invention relates to a method for editing two genes, specifically, a first complex of the Cas9 enzyme and a first gRNA and a second complex of the Cas9 enzyme and a second gRNA are introduced into the cell for gene editing.

In a specific embodiment, a third complex of the Cas9 enzyme, the first gRNA and the second gRNA are simultaneously introduced into the cell for gene editing.

In a specific embodiment, the first complex and the second complex are introduced into the cell successively for gene editing.

In the first complex or the second complex or the third complex, the molar ratio of the Cas9 enzyme and the gRNA is 1:1∼1:10, preferably 1:3∼1:5, more preferably 1: 4.

For example, in the first complex, the molar ratio of the Cas9 enzyme and the gRNA is 1:1∼1:10, preferably 1:3∼1:5, and more preferably 1:4. In the second complex, the molar ratio of the Cas9 enzyme and the gRNA is 1:1 to 1:10, preferably 1:3 to 1:5, and more preferably 1:4. In the third complex, the molar ratio of the Cas9 enzyme to the sum of the first gRNA and the second gRNA is 1:1 to 1:10, preferably 1:3 to 1:5, and more preferably 1:4.

Herein, the molar ratio refers to a ratio between the substance amount of the Cas9 enzyme and the gRNA, wherein the amount of the Cas9 enzyme or enzyme activity is calculated based on the Cas9 enzyme specification provided by the manufacturer, and the amount of corresponding gRNA is calculated according to composition of RNA bases and concentration of *in vitro* transcription.

In a specific embodiment, the ratio of the Cas enzyme and the gRNA is 1:4.

In a specific embodiment, the cell is a eukaryotic cell; in a specific embodiment, the eukaryotic cell is an immune effector cell; in a specific embodiment, the immune effector cell is a T cell.

In a specific embodiment, in the complex of the Cas enzyme and the gRNA, the concentration of the Cas enzyme is about 0.1µM∼3µM; preferably about 0.125µM∼3µM; more preferably about 0.2µM∼ 3µM; more preferably about 0.25µM to 3µM; more preferably about 0.5µM to 3µM.

In a specific embodiment, the complex formed by the Cas9 enzyme and the gRNA or the first complex or the second complex or the third complex, the concentration of the Cas9 enzyme is bout 0.1µM∼3µM; preferably about 0.125µM-3µM; more preferably about 0.2µM-3µM; more preferably about 0.25µM∼3µM; more preferably about 0.5µM∼3µM.

In a specific embodiment, in the complex of the Cas enzyme and the gRNA, the concentration of the Cas enzyme is about 0.1µM∼2 µM; preferably about 0.125µM∼2 µM; more preferably about 0.5µM∼ 2 µM; more preferably about 0.5µM to 2 µM; more preferably about 0.5µM to 2 µM.

In a specific embodiment, the cell is a T cell, and gene editing is performed on genes of T cell by the CRISPR/Cas system; in a specific embodiment, gene editing is performed on genes of any one or two of an α chain and a β chain of TCR of the T cell; in a specific embodiment, gene editing is performed on TRAC; in a specific embodiment, gene editing is performed on a constant region of the TRAC; in a specific embodiment, gene editing is performed on the sequence shown in SEQ ID NO:1 comprised in the TRAC.

In a specific embodiment, the cell is a T cell, and the CRISPR/Cas9 system is used for editing of a gene of the T cell; comprising:
using the CRISPR/Cas9 system to perform gene editing on either or both of the genes of the α chain and the β chain of the TCR of the T cell; preferably to perform gene editing on the TRAC; more preferably to perform gene editing on the constant region of the TRAC; more preferably to perform gene editing on the sequence shown in SEQ ID NO: 45 in the TRAC; more preferably to perform gene editing on the sequence shown in SEQ ID NO:1 comprised in the TRAC, and/or
using the CRISPR/Cas9 system to perform gene editing on a MHC gene of the T cell, preferably to perform gene editing on a B2M gene, more preferably to perform gene editing on the sequence shown in SEQ ID NO: 38 in the B2M gene, and more preferably to perform gene editing on the sequence shown in SEQ ID NO: 10 comprised in the B2M gene.

In a specific embodiment, the gRNA is designed according to a PAM sequence in the sequence shown in SEQ ID NO:1.

In a specific embodiment, the gRNA is about 15-50 bp, preferably about 15-30 bp, more preferably about 17-21 bp; more preferably 20 bp.

In a specific embodiment, the gRNA adopted for editing the TRAC includes the sequence shown in SEQ ID NO: 2, 3, 4, or 5; preferably, the gRNA used comprises the sequence shown in SEQ ID NO: 2.

In a specific embodiment, the gRNA adopted for editing the TRAC is the sequence shown in SEQ ID NO: 2, 3, 4, 5, 32, 33, 39 or 40; preferably, the gRNA adopted is the sequence shown in SEQ ID NO: 2, 32 or 33.

In a specific embodiment, the gRNA adopted for editing the TRAC is the sequence shown in SEQ ID NO: 2, 3, 4, 5, 32, 33, 39 or 40; preferably, the gRNA adopted is the sequence shown in SEQ ID NO: 2, 32 or 33.

Specifically, the aforementioned first gRNA may comprises the sequence shown in SEQ ID NO: 2, 3, 4, 5, 32, 33, 39, or 40.

In a specific embodiment, the concentration of the Cas enzyme is about 0.1µM to 0.5µM; preferably about 0.125µM to 0.5µM, more preferably about 0.25µM to 0.5µM.

In a specific embodiment, the cell is a T cell, and gene editing is performed on the B2M gene of the T Cell by the CRISPR/Cas system; in a specific embodiment, gene editing is performed on the sequence shown in SEQ ID NO: 10 comprised in the B2M gene; in a specific embodiment, the gRNA is designed according to the PAM sequence in the sequence shown in SEQ ID NO: 10.

In a specific embodiment, the gRNA adopted for editing the B2M gene comprises the sequence shown in SEQ ID NO: 11, 12, 13, or 14; preferably, the adopted gRNA comprises the sequence shown in SEQ ID NO: 12.

In a specific embodiment, the gRNA adopted for editing the B2M gene is the sequence shown in SEQ ID NO: 11, 12, 13, or 14; preferably, the adopted gRNA is the sequence shown in SEQ ID NO: 12.

Specifically, the aforementioned second gRNA may comprises the sequence shown in SEQ ID NO: 11, 12, 13, or 14.

Hereinafter, the descriptions for the first complex, the second complex or the third complex are consistent with the above, and the description for the first gRNA and the second gRNA are also consistent with the above. It should be understood that the complex, the first complex, the second complex or the third complex is intended to indicate different complexes, and there is no priority for their numbering. The same is for the first gRNA and the second gRNA that it is intended to indicate two different gRNAs, and one gRNA and another gRNA can also be used for indicating them, i.e., one gRNA can comprise the sequence shown in SEQ ID NO: 2, 3, 4, 5, 32, 33, 39 or 40, and the other gRNA can comprise the sequence shown in SEQ ID NO: 11, 12, 13, or 14.

In a specific embodiment, the concentration of the Cas enzyme is about 0.25µM to 3µM, preferably about 0.5µM to 3µM, and more preferably about 1µM to 3µM.

In a specific embodiment, the cell is a T cell, and gene editing is performed on the TRAC and B2M genes of the T cell by the CRISPR/Cas system; in a specific embodiment, gene editing is performed on first exons of the TRAC and B2M genes.

In a specific embodiment, gene editing is performed on the TRAC and/or B2M genes, and the TRAC and/or B2M genes are silenced.

In a specific embodiment, the gRNA adopted for editing the TRAC comprises the sequence shown in SEQ ID NO: 2, 3, 4, or 5, and the gRNA adopted for editing the B2M gene comprises the sequence shown in SEQ ID NO: 11, 12, 13, or 14; preferably, the gRNA adopted for editing the TRAC comprises the sequence shown in SEQ ID NO: 2, and the gRNA adopted for editing the B2M gene comprises the sequence shown in SEQ ID NO: 12.

In a specific embodiment, the gRNA is about 15-50 bp, preferably about 15-30 bp, more preferably about 20 bp; in a specific embodiment, it is 20 bp.

In a specific embodiment, when gene editing is performed on the TRAC and B2M genes, the ratio of B2M-editing gRNA and TRAC-editing gRNA adopted is about 1.5:1 to 0.5:1; preferably about 1:1. In a specific embodiment, the concentration of the Cas enzyme is about 1µM to 3µM.

In a specific embodiment, the T cell also expresses a chimeric receptor, an exogenous cytokine, an inhibitory/activating receptor or ligand, a costimulating factor; in a specific embodiment, the T cell further expresses a chimeric antigen receptor.

In the second aspect of the present invention, provided is a method for gene editing of TRAC gene of a T cell based on a CRISPR/Cas system. A complex of a Cas enzyme and a gRNA is introduced into the cell for gene editing, wherein the ratio of the Cas enzyme and the gRNA is 1:3∼1:5; in a specific embodiment, the Cas enzyme is a Cas9 enzyme.

In a specific embodiment, gene editing is performed on genes of any one or two of an α chain and a β chain of TCR of the T cell; in a specific embodiment, gene editing is performed on the TRAC of the T cell; in a specific embodiment, gene editing is performed on the constant region of the TRAC of the T cell; in a specific embodiment, gene editing is performed on the sequence shown in SEQ ID NO:1 comprised in the TRAC of the T cell; in a specific embodiment, the gRNA is designed according to a PAM sequence in the sequence shown in SEQ ID NO:1.

In a specific embodiment, the ratio of the Cas enzyme and the gRNA is 1:4.

In a specific embodiment, the concentration of the Cas enzyme is about 0.1µM to 0.5µM; preferably about 0.125µM to 0.5µM, more preferably about 0.25µM to 0.5µM.

In a specific embodiment, the gRNA adopted for editing the TRAC comprises the sequence shown in SEQ ID NO: 2, 3, 4, or 5; preferably, the gRNA adopted comprises the sequence shown in SEQ ID NO: 2.

In a specific embodiment, when editing the TRAC, the ratio of the Cas enzyme and the gRNA is 1:4; the concentration of the Cas enzyme is 0.25µM to 0.5µM; the gRNA comprises the sequence shown in SEQ ID NO: 2.

In the third aspect of the present invention, provided is a method for gene editing of a B2M gene of a T cell based on a CRISPR/Cas system. A complex of the Cas enzyme and the gRNA is introduced into the cell for gene editing, wherein the ratio of the Cas enzyme and the gRNA is 1:3∼1:5; in a specific embodiment, the Cas enzyme is a Cas9 enzyme.

In a specific embodiment, gene editing is performed on the sequence shown in SEQ ID NO: 10 comprised in the B2M gene.

In a specific embodiment, the gRNA is designed according to a PAM sequence in the sequence shown in SEQ ID NO:10. In a specific embodiment, the ratio of the Cas enzyme and the gRNA is 1:4.

In a specific embodiment, the concentration of the Cas enzyme is about 0.25µM to 3µM, preferably about 0.5µM to 3µM, and more preferably about 1µM to 3µM.

In a specific embodiment, the gRNA adopted for editing the B2M gene comprises the sequence shown in SEQ ID NO: 11, 12, 13, or 14; preferably, the gRNA adopted comprises the sequence shown in SEQ ID NO: 12.

In a specific embodiment, when editing the B2M gene, the ratio of the Cas enzyme and the gRNA is 1:4; the concentration of the Cas enzyme is 1µM∼3µM; the gRNA comprises the sequence shown in SEQ ID NO: 12.

In the fourth aspect of the present invention, provided is a method for gene editing of a TRAC gene and a B2M gene of a T cell based on a CRISPR/Cas system. A complex of a Cas enzyme and a gRNA is introduced into the cell, wherein the ratio of the Cas enzyme to the total gRNAs is 1:3∼1:5; in a specific embodiment, the Cas enzyme is a Cas9 enzyme.

In a specific embodiment, gene editing is performed on the sequence shown in SEQ ID NO: 10 comprised in the B2M gene; in a specific embodiment, in a specific embodiment, the gRNA is designed according to a PAM in the sequence shown in SEQ ID NO: 10.

In a specific embodiment, gene editing is performed on any one or twoof an α chain and a β chain of TCR; in a specific embodiment, gene editing is performed on TRAC;

In a specific embodiment, gene editing is performed on the constant region of the TRAC;

In a specific embodiment, gene editing is performed on the sequence shown in SEQ ID NO:1 comprised in TRAC; in a specific embodiment, in a specific embodiment, the gRNA is designed according to the PAM in the sequence shown in SEQ ID NO:1.

In a specific embodiment, the ratio of the Cas enzyme to the total gRNAs is 1:4. In a specific embodiment, the concentration of the Cas enzyme is 1µM to 3µM.

In a specific embodiment, the ratio of the gRNA used for editing the B2M gene and for editing the TRAC is 0.5:1 to 1.5:1, preferably 1:1.

In a specific embodiment, the gRNA adopted for editing the B2M gene comprises the sequence shown in SEQ ID NO: 11, 12, 13, or 14; preferably, the gRNA adopted comprises the sequence shown in SEQ ID NO: 12.

In a specific embodiment, the gRNA used for editing the TRAC comprises the sequence shown in SEQ ID NO: 2, 3, 4, or 5; preferably, the gRNA adopted comprises the sequence shown in SEQ ID NO: 2.

In a specific embodiment, the ratio of the Cas enzyme to the total gRNAs is 1:4; the concentration of the Cas enzyme is 1µM-3µM; the gRNA adopted comprises the sequence shown in SEQ ID NO: 12 and the sequence shown in SEQ ID NO: 2.

In a specific embodiment, the T cells described in the second, the third, and the fourth aspects above further expresses the chimeric receptor that recognizes a tumor antigen or a pathogen antigen, wherein the chimeric receptor has an extracellular antigen binding domain, a transmembrane domain, and an intracellular domain, and the extracellular antigen binding domain specifically recognizes the target antigen.

In a specific embodiment, the target antigen is a tumor antigen selected from the group consisting of: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3(CD276), B7H6; KIT (CD117); interleukin 13 receptor subunit α (IL - 13Rα); interleukin 11 receptor α (IL-11Rα); prostate stem cell antigen (PSCA); prostate specific membrane antigen (PSMA); carcinoembryonic antigen (CEA); NY-ESO-1; HIV-1 Gag; MART -1; gp100; tyrosinase; mesothelin; EpCAM; protease serine 21 (PRSS21); vascular endothelial growth factor receptor; Lewis (Y) antigen; CD24; platelet-derived growth factor receptor β (PDGFR-β); stage-specific embryonic antigen-4 (SSEA-4); cell surface-associated mucin 1 (MUC1), MUC6; epidermal growth factor 20 receptor family and its mutants (EGFR, EGFR2, ERBB3, ERBB4, EGFRvIII); nerve cell adhesion molecule (NCAM); carbonic anhydrase IX (CAIX); LMP2; ephrin A receptor 2 (EphA2); fucosyl GM1; sialyl Lewis adhesion molecule (sLe); O-acetyl GD2 ganglioside (OAcGD2); ganglioside GM3 (aNeu5Ac(2-3)bDGalp(1-4)bDGlcp(1-1)Cer; TGS5; high molecular weight melanoma-associated antigen (HMWMAA); folate receptor; tumor vascular endothelial marker 251 (TEM1/CD248); tumor vascular endothelial marker 7 related (TEM7R); Claudin6, Claudin18.2 (CLD18A2), Claudin18.1; ASGPR1; CDH16; 5T4; 8H9; αvβ6 integrin; B cell maturation antigen (BCMA); CA9; κ light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor, family C, group 5, member D (GPRC5D); X chromosome open reading frame 61 (CXORF61); CD97; CD179a; anaplastic lymphoma kinase (ALK); polysialic acid; placenta specific 1 (PLAC1); hexose part of globoH glycoceramide (GloboH); breast differentiation antigen (NY-BR-1); uroplakin 2 (UPK2); hepatitis A virus cell receptor 1 (HAVCR1); adrenaline receptor 5 β3 (ADRB3); pannexin 3 (PANX3); G protein coupled receptor 20 (GPR20); lymphocyte antigen 6 complex locus K9 (LY6K); olfactory receptor 51E2 (OR51E2); TCRγ alternating reading frame protein (TARP); Wilms tumor protein (WT1); ETS translocation variant gene 6 (ETV6-AML); sperm protein 17 (SPA17); X antigen family member 1A (XAGE1); angiopoietin binding cell-surface receptor 2 (Tie2); melanoma cancer testis antigen-1 (MAD-CT-1); melanoma cancer testis antigen-2 (MAD-CT-2); Fos-related antigen 1; p53 mutant 10; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin B1; V-myc avian myelocytomatosis virus oncogene neuroblastoma-derived homolog (MYCN); Ras homolog family member C(RhoC); Cytochrome P450 1B1 (CYP1B1); CCCTC binding factor (zinc finger protein)-like (BORIS); Squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte associated immunoglobulin like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1).

In a specific embodiment, the target antigen is a pathogen antigen, and the pathogen antigen is selected from the group consisting of: virus, bacteria, fungus, protozoa, or parasite antigen; in one embodiment, the virus antigen is selected from the group consisting of cytomegalovirus antigen, Epstein-Barr virus antigen, human immunodeficiency virus antigen or influenza virus antigen.

In a specific embodiment, the chimeric receptor is selected from the group consisting of: a chimeric antigen receptor (CAR) or a T cell antigen coupler (TAC).

In a specific embodiment, the chimeric receptor is a chimeric antigen receptor. In a specific embodiment, the chimeric antigen receptor comprises:
(i) an antibody that specifically binds to a tumor antigen, a transmembrane region of CD28 or CD8, a costimulatory signaling domain of CD28, and CD3ζ; or
(ii) an antibody that specifically binds to a tumor antigen, the transmembrane region of CD28 or CD8, the costimulatory signaling domain of CD137, and CD3ζ; or
(iii) an antibody that specifically binds to a tumor antigen, the transmembrane region of CD28 or CD8, the costimulatory signaling domain of CD28, the costimulatory signaling domain of CD137, and CD3ζ.

In a specific embodiment, the chimeric receptor is TAC, comprising:
(a) an extracellular domain: the extracellular domain comprises an antibody domain having an antigen-binding domain, and a single-chain antibody that binds to CD3;
(b) a transmembrane domain;
(c) an intracellular domain, which is connected to a protein kinase LCK.

In a specific embodiment, the antibody of the chimeric antigen receptor that specifically binds to a tumor antigen is a full-length antibody, scFv, Fab, (Fab'), or single domain antibody.

In the fifth aspect of the present invention, the use of the T cells described in the second, the third, and the fourth aspects above is provided, for preparing a chimeric receptor-expressing T cell, the chimeric receptor has an extracellular antigen binding domain, a transmembrane domain, and an intracellular domain, wherein, the extracellular antigen binding domain specifically recognizes a target antigen.

In a specific embodiment, the target antigen is a tumor antigen or a pathogen antigen.

In a specific embodiment, the target antigen is a tumor antigen selected from the group consisting of: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3(CD276), B7H6; KIT (CD117); interleukin 13 receptor subunit α (IL - 13Rα); interleukin 11 receptor α (IL-11Rα); prostate stem cell antigen (PSCA); prostate specific membrane antigen (PSMA); carcinoembryonic antigen (CEA); NY-ESO-1; HIV-1 Gag; MART -1; gp100; tyrosinase; mesothelin; EpCAM; protease serine 21 (PRSS21); vascular endothelial growth factor receptor; Lewis (Y) antigen; CD24; platelet-derived growth factor receptor β (PDGFR-β); stage-specific embryonic antigen-4 (SSEA-4); cell surface-associated mucin 1 (MUC1), MUC6; epidermal growth factor 20 receptor family and its mutants (EGFR, EGFR2, ERBB3, ERBB4, EGFRvIII); nerve cell adhesion molecule (NCAM); carbonic anhydrase IX (CAIX); LMP2; ephrin A receptor 2 (EphA2); fucosyl GM1; sialyl Lewis adhesion molecule (sLe); O-acetyl GD2 ganglioside (OAcGD2); ganglioside GM3 (aNeu5Ac(2-3)bDGalp(1-4)bDGlcp(1-1)Cer; TGS5; high molecular weight melanoma-associated antigen (HMWMAA); folate receptor; tumor vascular endothelial marker 251 (TEM1/CD248); tumor vascular endothelial marker 7 related (TEM7R); Claudin6, Claudin18.2 (CLD18A2), Claudin18.1; ASGPR1; CDH16; 5T4; 8H9; ανβ6 integrin; B cell maturation antigen (BCMA); CA9; κ light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor, family C, group 5, member D (GPRC5D); X chromosome open reading frame 61 (CXORF61); CD97; CD179a; anaplastic lymphoma kinase (ALK); polysialic acid; placenta specific 1 (PLAC1); hexose part of globoH glycoceramide (GloboH); breast differentiation antigen (NY-BR-1); uroplakin 2 (UPK2); hepatitis A virus cell receptor 1 (HAVCR1); adrenaline receptor 5 β3 (ADRB3); pannexin 3 (PANX3); G protein coupled receptor 20 (GPR20); lymphocyte antigen 6 complex locus K9 (LY6K); olfactory receptor 51E2 (OR51E2); TCRγ alternating reading frame protein (TARP); Wilms tumor protein (WT1); ETS translocation variant gene 6 (ETV6-AML); sperm protein 17 (SPA17); X antigen family member 1A (XAGE1); angiopoietin binding cell-surface receptor 2 (Tie2); melanoma cancer testis antigen-1 (MAD-CT-1); melanoma cancer testis antigen-2 (MAD-CT-2); Fos-related antigen 1; p53 mutant 10; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin B1; V-myc avian myelocytomatosis virus oncogene neuroblastoma-derived homolog (MYCN); Ras homolog family member C(RhoC); Cytochrome P450 1B1 (CYP1B1); CCCTC binding factor (zinc finger protein)-like (BORIS); Squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte associated immunoglobulin like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1).

In a specific embodiment, the target antigen is a pathogen antigen, and the pathogen antigen is selected from the group consisting of virus antigen, bacteria antigen, fungus, protozoa, or parasite antigen; in one embodiment, the virus antigen is selected from the group consisting of cytomegalovirus antigen, Epstein-Barr virus antigen, human immunodeficiency virus antigen or influenza virus antigen.

In a specific embodiment, the chimeric receptor is selected from the group consisting of: a chimeric antigen receptor (CAR) or a T cell antigen coupler (TAC).

In a specific embodiment, the chimeric receptor is a chimeric antigen receptor (CAR).

In a specific embodiment, the chimeric antigen receptor comprises:
(i) an antibody that specifically binds to a tumor antigen, a transmembrane region of CD28 or CD8, a costimulatory signaling domain of CD28, and CD3ζ; or
(ii) an antibody that specifically binds to a tumor antigen, a transmembrane region of CD28 or CD8, a costimulatory signaling domain of CD137, and CD3ζ; or
(iii) an antibody that specifically binds to a tumor antigen, a transmembrane region of CD28 or CD8, a costimulatory signaling domain of CD28, a costimulatory signaling domain of CD137, and CD3ζ.

In a specific embodiment, the chimeric receptor is TAC, comprising:
(a) an extracellular domain: the extracellular domain comprises an antibody domain having an antigen-binding domain, and a single-chain antibody that binds to CD3;
(b) a transmembrane domain;
(c) an intracellular domain, which is connected to a protein kinase LCK.

In a specific embodiment, the antibody of the chimeric antigen receptor that specifically binds to a tumor antigen is a full-length antibody, scFv, Fab, (Fab'), or single domain antibody.

In the present invention, there is no specific limitation on electrotransfection conditions. For example, the electrotransfection conditions may be 150-600V, 0.5ms-20ms, for example, preferably be 150V-300V, 2ms-15ms.

In a specific embodiment, the molar ratios of the gRNA for gene editing of the TCR gene and the gRNA for gene editing of the MHC gene is about 1:5 to 5:1, preferably 1:2 to 2:1; more preferably about 1:1.

In a specific embodiment, the T cell is as shown in the above aspects.

In a specific embodiment, the chimeric receptor is a chimeric antigen receptor (CAR), and the chimeric antigen receptor is as shown in the above aspects..

In the seventh aspect of the present invention, it relates to a universal T cell, which is constructed according to the above-mentioned method of the present invention.

In the eighth aspect of the present invention, it relates to a universal T cell, wherein the TRAC and/or B2M genes are silenced.

In a specific embodiment, the TRAC gene is silenced by gene editing a sequence comprising the sequence shown in SEQ ID NO:1, and more preferably, the TRAC gene is silenced by gene editing the sequence shown in SEQ ID NO: 45 in the sequence comprising the sequence shown in SEQ ID NO: 1;
the B2M gene is silenced by gene editing a sequence comprising the sequence shown in SEQ ID NO:10, and more preferably, the B2M gene is silenced by gene editing the sequence shown in SEQ ID NO: 38 in the sequence comprising the sequence shown in SEQ ID NO: 10.

In a specific embodiment, the TRAC gene is silenced by gene editing the TRAC gene using the gRNA of the sequence shown in SEQ ID NO: 2, 32 or 33, the B2M gene is silenced by gene editing the B2M gene using the gRNA of the sequence shown in SEQ ID NO: 12.

In a specific embodiment, the T cell further expresses a chimeric antigen receptor, preferably the T cell further expresses a chimeric receptor that recognizes a tumor antigen or a pathogen antigen, the chimeric receptor has an extracellular antigen binding domain, a transmembrane domain, and an intracellular domain, and the extracellular antigen binding domain specifically recognizes a target antigen.

The T cell is as shown in the above aspects. The chimeric antigen receptor is as shown in the above aspects.

In the ninth aspect of the present invention, it relates to a gRNA construct comprising a nucleotide sequence selected from the group consisting of SEQ ID NO: 2, 3, 4, 5, 32, 33, 39, 40, 11, 12, 13, or 14.

In a specific embodiment, the gRNA construct of the present invention comprises: a nucleotide sequence selected from the group consisting of SEQ ID NO: 2, 3, 4, 5, 32, 33, 39, or 40, and a nucleotide sequence selected from the group consisting of SEQ ID NO: 11, 12, 13, or 14 sequence.

In a specific embodiment, the gRNA construct of the present invention comprises: a sequence selected from the group consisting of that shown in SEQ ID NO: 2, 32 or 33, and/or a sequence shown in SEQ ID NO: 12.

The present invention relates to the use of gene editing technology in the modification of T cells, which can effectively inhibit the functions of T cell antigen receptor (TCR) and major histocompatibility complex (MHC) in T cells through the knock-out of multiple genes, wherein the TCR encoding gene is TRAC, and the B2M encoding gene is MHC I. Based on the Cas9/CRISPR gene technology and improvement and optimization of an electrotransfection method of an RNP (a complex of a RNA nucleic acid and a protein), the one-time and efficient double knockout of the TRAC and B2M genes in a short time is realized, with a knockout efficiency of over 90%.

### BRIEF DESCRIPTION OF THE DRAWINGS

Figure 1 is a schematic diagram showing the binding sites of sgRNAs to the TRAC gene;
Figure 2 shows the effects of different composition ratios of RNP on the knockout efficiency of TRAC;
Figure 3 shows the effects of different gRNA sequences on the knockout efficiency of the TRAC;
Figure 4 shows the effects of different concentrations of Cas9 enzymes on the knockout efficiency of TRAC;
Figure 5 shows a schematic diagram of the binding sites of gRNAs to the B2M gene;
Figure 6 shows the effects of different gRNAs on the knockout efficiency of the B2M gene;
Figure 7 shows the effects of different concentrations of Cas9 enzymes on the knockout efficiency of the B2M gene;
Figure 8 shows, when simultaneously knocking out the TRAC and B2M, the effects of different gRNA components on the double knockout of the TRAC and B2M;
Figure 9 shows the effects of the concentrations of the RNP complex formed by the mixture composed of the TRAC and B2M genes-targeting gRNAs and Cas9 enzymes on the knockout efficiency;
Figure 10(a)-(d) shows the mutation efficiencies of TRAC and B2M genes predicted by the online software Tide;
Figure 11 shows the results of the TRAC and B2M gene mutations verified by sequencing the clones.
Figure 12 shows the gene knockout efficiencies of the TRAC and B2M genes in BCMA-targeting CAR T cells.

### DETAIL DESCRIPTION OF THE INVENTION

The inventor found that when gene editing is performed using the CRISPR/Cas9 system, the choice of gRNAs, the ratio of Cas9 enzymes and gRNAs, etc., have a great influence on the editing efficiency. On this basis, the present invention is accomplished.

### The terms

Unless specifically defined, all technical and scientific terms used herein have the same meanings commonly understood by those skilled in the fields of gene therapy, biochemistry, genetics, and molecular biology. All methods and materials similar or equivalent to those described herein can be used in the practice or testing of the present invention, among which methods and materials described herein are suitable. All publications, patent applications, patents and other references mentioned herein are entirely incorporated herein by reference. In case of conflict, the specification, including the definitions, will control. In addition, unless otherwise specified, the materials, methods, and examples are illustrative only and not intended to be limiting.

Unless otherwise specified, traditional techniques of cell biology, cell culture, molecular biology, transgenic biology, microbiology, recombinant DNA and immunology will be adopted in the practice of the present invention, all of which fall within the technical scope of the art. These techniques are fully explained in the literature. See, for example, Current Protocols in Molecular Biology (FrederickM.AUSUBEL, 2000, Wileyand sonInc, Library of Congress, USA); Molecular Cloning: A Laboratory Manual, Third Edition, (Sambrooketal, 2001, Cold Spring Harbor, NewYork: Cold Spring Harbor Laboratory Press); Oligonucleotide Synthesis (M.J.Gaited. , 1984) ; Mullis et al. U.S. Pat. No. 4,683,195 ; Nucleic Acid Hybridization (B. D. Harries & S. J. Higginseds. 1984) ; Transcription And Translation (B. D. Hames & S. J. Higginseds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson 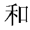 M. Simon, eds.-in-chief, Academic Press, Inc., New York), especially Vols.154 and 155 (Wuetal. eds.) and Vol.185, "Gene Expression Technology" (D. Goeddel, ed.); Gene Transfer Vectors For Mammalian Cells (J. H. Miller and M. P. Caloseds., 1987, Cold Spring Harbor Laboratory); Immunochemical Methods In Cell And Molecular Biology (Mayer and Walker, eds., Academic Press, London, 1987); Hand book Of Experimental Immunology, Vols.I-IV (D. M. Weir and C. C. Blackwell, eds., 1986); and Manipulating the Mouse Embryo (Cold Spring Harbor Laboratory Press, Cold Spring Harbor, N.Y., 1986). In the disclosure, all aspects of the claimed subject matters are presented in the form of range. It should be understood that the description in range format is merely for convenience and brevity, and should not be construed as an inflexible limitation on the scope of the claimed subject matter. Therefore, the description of a range should be considered to have specifically disclosed all possible subranges as well as individual values within the range. For example, in the case of providing a range of values, it should be understood that each intermediate value between the upper limit and the lower limit of the range and any other stated or intermediate values within the range are included in the claimed subject, so do the upper and lower limits of the range. The upper and lower limits of these smaller ranges may be independently included in the smaller range, and they also belong to the scope of the claimed subject matter, unless the upper and lower limits of the range are explicitly excluded. When the set range comprises one or two limit values, the claimed subject matter also comprises the ranges that exclude one or two of the limit values. This applies regardless of the width of the range.

The term about used herein refers to the usual error range of each value easily known to those skilled in the art. The reference to "about" a value or a parameter herein includs (and describes) an embodiment that refers to the value or the parameter itself. For example, the description of "about X" includes the description of "X". For example, "about" or "comprise" may mean within 1 or more than 1 according to the actual standard deviation in the field. Or "about" or "comprise" can mean a range of up to 10% (i.e., ±10%). For example, about 5µM can include any number between 4.5 µM and 5.5 µM. When a specific value or composition is provided in the application and the scope of the patent application,
unless otherwise indicated, "about" or "comprise" should be assumed to be within the acceptable error range of the particular value or composition.

Any concentration range, percentage range, ratio range or integer range described herein should be understood to include any integer within the stated range, and where appropriate, its fractions (for example, one-tenth and one-hundredth of an integer), unless otherwise indicated.

To facilitate a better understanding of the present invention, related terms are defined as follows:
The term "gene editing" refers to the ability to allow humans to "edit" target genes, achieving the knockout and addition of specific DNA fragments.

The term "molecular silencing" or "gene silencing" refers to the phenomenon that genes are not expressed or underexpressed due to various reasons without damaging the original DNA. Gene silencing occurs at two levels, one is gene silencing at the transcriptional level caused by DNA methylation, heterochromatinization, and positional effects, and the other is post-transcriptional gene silencing, i.e., at the level of gene transcription, genes are inactivated by specific inhibition of target RNA, including antisense RNA, co-suppression, gene suppression, RNA interference, microRNA mediated translational inhibition, and the like. CRISPR (Clustered regularly interspaced short palindromic repeats); Cas9 (CRISPR associated nuclease) is a CRISPR-associated nuclease, and CCRISPR/Cas9 is the latest RNA-guided technique using Cas9 nuclease for editing target genes.

"CRISPER/Cas9 system" is collectively referred to as transcripts and other elements involved in the expression of Cas9 enzyme genes or directing its activity, including sequences encoding Cas9 genes, tracr (trans-activating CRISPR) sequences (e.g., tracrRNA or the active part of tracrRNA), tracr pairing sequences (encompassing "direct repeats" and partial direct repeats of tracrRNA processing in the context of endogenous CRISPR systems), guide sequences (also called "spacers" in the context of endogenous CRISPR systems, i.e., gRNAs), or other sequences and transcripts from the CRISPR locus. Generally speaking, the CRISPR system is characterized by elements that promote the formation of a CRISPR complex (also referred to as an protospacer in the context of the endogenous CRISPR system) at the site of the target sequence..

The term "target sequence" refers to a sequence that has complementarity with a guide sequence. The complementary pairing between the target sequence and the guide sequence promotes the formation of a CRISPR complex. Complete complementarity is not required, provided that there is sufficient complementarity to cause hybridization and to promote the formation of a CRISPR complex. A target sequence can comprise any polynucleotide, such as DNA or RNA polynucleotide. In some embodiments, the target sequence is located in the nucleus or cytoplasm of the cell.

In general, a guide sequence (gRNA) is any polynucleotide sequence that has sufficient complementarity with a target polynucleotide sequence to hybridize with the target sequence and direct the sequence-specific binding of the CRISPR complex to the target sequence. In some embodiments, when a suitable alignment algorithm is used for optimal alignment, the degree of complementarity of the guide sequence and its corresponding target sequence is about or more than about 50%, 60%, 75%, 80% , 85%, 90%, 95%, 97.5%, 99%, or more. Any suitable algorithm for aligning sequences can be used to determine the optimal alignment, non-limiting examples of which include the Smith-Waterman algorithm, the Needleman-Wunsch algorithm, Algorithms based on the Burrows-Wheeler Transform (e.g., Burrows Wheeler Aligner), ClustalW, Clustal X, BLAT, Novoalign (Novocraft Technologies), ELAND Company (Illumina, San Diego, California), SOAP (available at soap.genomics.org.cn), and Maq (available at maq.sourceforge.net).

In some embodiments, the CRISPR enzyme is a part of the fusion protein comprising one or more heterologous protein domains (e.g., about or more than about 1, 2, 3, 4, 5, 6,7, 8, 9, 10 or more domains besides the CRISPR enzyme). The CRISPR enzyme fusion protein can comprise any other proteins, and optionally a linking sequence between any two domains. Examples of protein domains that can be fused to CRISPR enzymes include, but are not limited to, epitope tags, reporter gene sequences, and protein domains having one or more of the following activities: methylase activity, demethylase activity, transcription activation activity, transcription repression activity, transcript release factor activity, histone modification activity, RNA cleavage activity and nucleic acid binding activity. Non-limiting examples of epitope tags include histidine (His) tags, V5 tags, FLAG tags, influenza virus hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT), β-galactosidase, β-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). The CRISPR enzyme can be fused to a gene sequence encoding a protein or a protein fragment that binds to a DNA molecule or other cellular molecule, including, but not limited to, maltose binding protein (MBP), S-tag, Lex A DNA binding domain (DBD) fusion, GAL4 DNA binding domain fusion, and herpes simplex virus (HSV) BP16 protein fusion. Additional domains that can form a part of a fusion protein containing a CRISPR enzyme are described in US 20110059502, which is incorporated herein by reference.

The term "Cas9 enzyme" can be wild-type Cas9 or any modified version of Cas9, including any naturally occurring bacterial Cas9 and any chimera, mutant, homolog or ortholog. The Cas9 enzyme can comprise one or more mutations and can be used as a universal DNA binding protein with or without fusion to a functional domain. These mutations can be artificially introduced mutations or acquired and lost functional mutations. These mutations may include, but are not limited to, mutations in one of the catalytic domains (D10 and H840) in the RuvC and HNH catalytic domains, respectively.

In the present invention, for example, the Cas9 enzyme from NEB Company can be used. Of course, those skilled in the art can select other Cas9 enzymes with the same or similar functions. In this article, the function that the Cas9 enzyme can achieve is that, in a 30 µl of a reacted Cas9 enzyme reaction system (the reaction system including: 20 mM HEPES, 100 mM NaCl, 5 mM MgCl₂, 0.1 mM EDTA and at 25°C, pH is 6.5), under the condition that 1 nM PvuII linearized pBR322 DNA (with a target site CGCTTGTTTCGGCGTGGGTA), 40 nM sgRNA and 20 nM of the Cas9 enzyme are contained, in the case of incubation at 37°C for 1 hour, 90% of pBR322 DNA is comfirmed to be degraded through agarose gel electrophoresis. In this reaction system, the amount of the Cas9 enzyme that catalyzes the complete conversion of 1 nmol substrate (pBR322 DNA linearized by PvuII) into products in 1 minute is 0.37 nmol, and the amount of the Cas9 enzyme is 59.57ng. The enzyme activity of the Cas9 enzyme is 0.37 nmol (the amount of enzyme that catalyzes the conversion of 1 nmol of substrate into products in 1 minute).

Those skilled in the art can understand that the calculation of the molar ratio of the Cas9 enzymes and gRNAs desired to be introduced and the determination of the concentration of the Cas9 enzymes in the introduced complex are based on the above Cas9 enzyme activity herein. When the activity of the Cas9 enzyme changes, those skilled in the art can select the concentration of the Cas9 enzymes and its molar ratio to gRNAs by conversion based on the ratio determined herein and according to the description of the activity in the specifications of different enzymes.

In one aspect, the Cas enzyme is a nicking enzyme. In a preferred embodiment, the Cas9 is delivered to a cell in the form of mRNA, allowing transient expression of the enzyme, thereby reducing the toxicity. Cas9 can also be delivered to cells in a nucleotide construct that encodes and expresses the Cas9 enzyme. In addition, Cas9 can also be expressed under the control of an inducible promoter.

The terms CRISPR and Cas enzyme are generally used interchangeably herein, unless otherwise stated. As mentioned above, many residue numbers used herein refer to that of the Cas9 enzyme from the type II CRISPR locus in *Streptococcus pyogenes.* However, it should be understood that the present invention comprises more Cas9 from other microbial species, such as SpCas9, SaCa9, St1Cas9, etc. Those skilled in the art will be able to determine the appropriate corresponding residues in Cas9 enzymes other than SpCas9 by comparing related amino acid sequences. The term sgRNA refers to a short gRNA. When performing gene editing, the given gRNA, tracr pairing sequence, and tracr sequence can be given separately, or be given in a integrated RNA sequence. The binding of Cas9 protein and the gRNA can realize the cleavage of DNA at specific sites. The CRISPR/Cas system recognition sequence derived from *Streptococcus pyogenes* is 23bp and can target 20bp. The sequence of the last 3 nucleotides (NGG) in its recognition site is called a PAM(protospacer adjacent motif) sequence.

Unless otherwise indicated, the terms Cas enzyme, CRISPR enzyme, CRISPR protein, Cas protein and CRISPR Cas are generally used interchangeably.

The Cas transgene can be delivered by vectors (e.g., AAV, adenovirus, lentivirus), and/or particles and/or nanoparticles, and/or electrotransfection.

In one embodiment, the exons of the corresponding coding genes in the constant regions of one or two of an α chain and a β chain of TCR are knocked out using the CRISPR/Cas technology to inactivate the endogenous TCR. Preferably, the first exon of the constant region of the endogenous TCRα chain is targeted to be knocked out.

"Inhibiting" or "suppressing" the expression of B2M or TCR means that the expression of the B2M or TCR in a cell is reduced by at least 1%, at least 5%, at least 10%, at least 20%, at least 30%, at least 40%, at least 50%, at least 60%, at least 70%, at least 80%, at least 90%, at least 95%, at least 99%, or 100%. More specifically, "inhibiting" or "suppressing" the expression of the B2M means that the content of the B2M in the cell is reduced by at least 1%, at least 5%, at least 10%, at least 20%, at least 30%, at least 40%, at least 50%, at least 60%, at least 70%, at least 80%, at least 90%, at least 95%, at least 99% or 100%. The expression or content of a protein in cells can be determined by any suitable method known in the art, such as ELISA, immunohistochemistry, Western Blotting or flow cytometry, using specific antibodies of the B2M or TCR.

The term "modification" used in the present invention refers to a change in the state or structure of the protein or polypeptide of the present invention. Modification methods can be chemical, structural and functional. T cell receptor (TCR) is a cell surface receptor that participates in T cell activation in response to antigen presentation. TCR is usually composed of two chains, α and β, which can assemble to form a heterodimer and associate with a CD3 transducing subunit to form a T cell receptor complex present on the cell surface. The α and β chains of the TCR are composed of the following: immunoglobulin-like N-terminal variable regions (V) and constant regions (C), hydrophobic transmembrane domains and short cytoplasmic regions. For immunoglobulin molecules, the variable regions of the α chain and the β chain are produced by V(D)J recombination, resulting in the production of a large number of diverse antigen specificities in the population of T cells. However, in contrast to immunoglobulins that recognize complete antigens, T cells are activated by processed peptide fragments associated with MHC molecules, and additional dimensions are introduced into antigen recognition by T cells, which is called MHC restriction. Recognizing the MHC difference between a donor and a recipient by the T cell receptor leads to cell proliferation and the potential development of GVHD. It has been shown that the normal surface expression of TCR depends on the synergistic synthesis and assembly of all seven components of a complex (Ashwell and Klusner 1990). The inactivation of TCRα or TCRβ can lead to the elimination of TCR from the surface of T cells, thereby preventing the recognition of allogeneic antigens and the resulting GVHD.

The term "MHC" is the histocompatibility complex, which is a general term for the group of all genes encoding the biocompatibility complex antigens. MHC antigens are expressed in the tissues of all higher vertebrates and are called HLA antigens in human cells. MHC antigens play an important role in transplantation reactions, as rejection is mediated by T cells that response to the histocompatibility antigens on the surface of the implanted tissue. MHC proteins play a vital role in T cell stimulation. Antigen-presenting cells (usually dendritic cells) display peptides that belong to the degradation products of foreign proteins on the cell surface on MHC. In the presence of costimulatory signals, T cells are activated and act on target cells that also display the same peptide/MHC complex. For example, stimulated T helper cells will target macrophages that display antigens bound to their MHC; or cytotoxic T cells (CTL) will act on virus-infected cells that display foreign viral peptides. MHC antigens are divided into NHC class I antigens and MHC class II antigens. In humans, the class I HLA gene cluster includes three major loci HLA-A, HLA-B, and HLA-C, as well as several minor loci. The Class II HLA cluster also includes three main loci: HLA-DP, HLA-DQ and HLA-DR,

The term "human leukocyte antigen" (Human leukocyte antigen, HLA) is the human major histocompatibility complex coding gene, located on chromosome 6 (6p21.31), including a series of closely linked loci, and closely related to the function of human immune system. HLA includes class I, class II, and class III gene parts. The antigens expressed by HLA class I and class II genes are located on the cell membrane, and are MHC-I (encoded by HLA-A, HLA-B, HLA-C sites) and MHC-II (encoded by HLA-D region). Class I distributed on the surface of almost all cells in the body. It is a heterodimer composed of a heavy chain (α chain) and a β2 microglobulin (B2M). Type II is mainly glycoprotein located on the surface of macrophages and B lymphocytes.

The term "B2M" refers to β-2 microglobulin, also known as B2M, which is the light chain of MHC class I molecules. In humans, B2M is encoded by the b2m gene located on chromosome 15, as opposed to other MHC genes located as gene clusters on chromosome 6. A mouse model of β-2 microglobulin deficiency indicates that B2M is necessary for the cell surface expression of MHC class I and the stability of the peptide binding groove. It further showed that due to targeted mutations in the β-2 microglobulin gene, hematopoietic grafts from mice lacking normal cell surface MHC I expression are rejected by NK1.1+ cells in normal mice, indicating that the defective expression of MHC I molecules makes bone marrow cells easily rejected by the host immune system (Bix et al. 1991).

Therefore, in order to provide T cells with lower allogeneic reactivity, the T cells provided by the present invention comprise T cells that have inactivated or mutated one TCR gene and one HLA gene.

The "inactive TCR" means an endogenous TCR with at least one inactive subunit gene, especially inactive TCRα and/or TCRβ genes, and more preferably, the TCRα gene.

The " inactive MHC " means an endogenous MHC with at least one inactive subunit gene, especially inactive MHC I gene, and more preferably, B2M gene.

The term "T cell antigen coupler (TAC)" includes three functional domains: a tumor targeting domain, including a single-chain antibody, designed ankyrin repeat protein (DARPin) or other targeting group 2, which is the domain of the extracellular region and a single-chain antibody binding to CD3, so that the TAC receptor is close to the other TCR receptors; a transmembrane region; and the intracellular region of a CD4 co-receptor; wherein the intracellular region is connected to the protein kinase LCK, which catalyzes the phosphorylation of immunoreceptor tyrosine activation motifs (ITAMs) of the TCR complex, acting as the initial step of T cell activation.

As used herein, the terms "stimulate" and "activate" are used interchangeably, and they and other grammatical forms thereof can refer to the process by which a cell changes from a resting state to an active state. The process may include a response to antigen, migration, and/or phenotypic or genetic changes of functional activity status. For example, the term "activation" can refer to the process of gradual activation of T cells. For example, T cells may require at least two signals to be fully activated. The first signal can occur after the binding of TCR to the antigen-MHC complex, and the second signal can occur through the binding of costimulatory molecules (see the costimulatory molecules listed in Table 1). In vitro, anti-CD3 can simulate the first signal, and anti-CD28 can simulate the second signal. For example, engineered T cells can be activated by expressed CAR. T cell activation or T cell triggering as used herein may refer to the state that T cells have been sufficiently stimulated to induce detectable cell proliferation, cytokine production, and/or detectable effector function.

The term "chimeric receptor" refers to a fusion molecule formed by linking DNA fragments or cDNAs corresponding to proteins from different sources using gene recombination technology, comprising an extracellular domain, a transmembrane domain and an intracellular domain. Chimeric receptors include but are not limited to: chimeric antigen receptor (CAR), modified T cell (antigen) receptor (TCR), T cell fusion protein (TFP), and T cell antigen coupler (TAC).

The term "costimulatory ligand" includes molecules on antigen-presenting cells (for example, aAPC, dendritic cells, B cells, etc.) that specifically bind to identical costimulatory molecules on T cells , thereby providing a signal. Together with the first signal provided by, for example, the combination of the TCR/CD3 complex and the peptide-loaded MHC molecule, it mediates the T cell response, including but not limited to proliferation, activation, differentiation, and the like.. Costimulatory ligand may include, but is not limited to, CD7, B7-1 (CD80), B7-2 (CD86), PD-L, PD-L2, 4-1BBL, OX40L, and inducible costimulatory ligand (ICOS-L), intercellular adhesion molecule (ICAM), CD30L, CD40, CD70, CD83, HLA-G, MICA, MICB, HVEM, lymphotoxin β receptor, 3/TR6, ILT3, ILT4, HVEM, agonists or antibodies that bind the toll ligand receptor and ligands that specifically bind to B7-H3. Costimulatory ligands also specifically include antibodies that specifically bind to costimulatory molecules present on T cells, for example but not limited to CD27, CD28, 4-1BB, OX40, CD30, CD40, PD-1, ICOS, lymphocyte function related antigen-1 (LFA-1), CD2, CD7, LIGHT, NKG2C, B7-H3 and ligands that specifically bind to CD83.

The term "costimulatory molecule" refers to the identical binding partner on a T cell that specifically binds to a costimulatory ligand, thereby mediating a costimulatory response of the T cell, such as but not limited to proliferation. Costimulatory molecules include but are not limited to MHC class I molecules, BTLAs and Toll ligand receptors.

The term "costimulatory signal" refers to a signal, by combining with cell stimulatory signal molecules, such as the TCR/CD3 combination, leads to T cell proliferation and/or up- or down-regulation of key molecules.

The term "chimeric antigen receptor" or "CAR" refers to an engineered molecule that can be expressed by immune cells including but not limited to T cells. CAR is expressed in T cells and can redirect T cells to induce the killing of target cells with specificity determined by artificial receptors. The extracellular binding domain of CAR can be derived from murine, humanized or fully human monoclonal antibodies. When it is in immune effector cells, it provides the cells with specificity for target cells (usually cancer cells) and has intracellular signal production. CAR usually comprises at least an extracellular antigen binding domain, a transmembrane domain and a cytoplasmic signaling domain (also referred to herein as "intracellular signaling domain"), which includes stimulatory molecules and/or the functional signaling domains of costimulatory molecules derived from the following definitions. In certain aspects, groups of polypeptides are adjacent to each other. The group of polypeptides includes dimerization switches that can couple polypeptides to each other in the presence of a dimerization molecule, for example, antigen binding domains can be coupled to an intracellular signaling domain. In one aspect, the stimulatory molecule is the ζ chain that binds to the T cell receptor complex. In one aspect, the cytoplasmic signaling domain further comprises one or more functional signaling domains derived from at least one costimulatory molecule as defined below. In one aspect, the costimulatory molecule is selected from the group consisting of costimulatory molecules described herein, such as 4-1BB (i.e., CD137), CD27, and/or CD28. In one aspect, the CAR comprises a chimeric fusion protein which comprises an extracellular antigen binding domain, a transmembrane domain, and an intracellular signaling domain comprising a functional signaling domain derived from a stimulatory molecule. In one aspect, the CAR comprises a chimeric fusion protein which comprises an extracellular antigen-binding domain, a transmembrane domain, and an intracellular signaling domain comprising a functional signaling domain derived from a costimulatory molecule and a functional signaling domain derived from a stimulatory molecule. In one aspect, the CAR comprises a chimeric fusion protein comprising an extracellular antigen binding domain, a transmembrane domain, and two functional signaling derived from one or more costimulatory molecules.

The term "signaling domain" refers to a functional part of a protein that functions by transmitting information in a cell, and is used to regulate the cell activity through a defined signaling pathway by producing a second messenger or act as an effector responding to the messenger.

The term "cell" and other grammatical forms thereof can refer to a cell of human or non-human animal origin. Engineered cells can also refer to cells expressing CAR.

The term "transfection" refers to the introduction of exogenous nucleic acid into eukaryotic cells. Transfection can be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retrovirus infection and biolistics.

The term "stable transfection" or "stably transfecting" refers to the introduction and integration of exogenous nucleic acid, DNA or RNA into the genome of the transfected cell. The term "stable transfectant" refers to a cell that stably integrates foreign DNA into the genomic DNA.

The terms "nucleic acid molecule code", "encoding DNA sequence" and "encoding DNA" refer to the sequence or the order of deoxyribonucleotides along a deoxyribonucleic acid chain. The order of the deoxyribonucleotides determines the order of amino acids along a polypeptide (protein) chain. Therefore, the nucleic acid sequence encodes an amino acid sequence.

The term "subject" refers to any animal, for example a mammal or marsupial. Subjects of the present invention include, but are not limited to, humans, non-human primates (such as rhesus monkeys or other types of macaques), mice, pigs, horses, donkeys, cattle, sheep, rats, and any kind of poultry.

The term "peripheral blood mononuclear cell" (PBMC) refers to cells with mononuclear nuclei in peripheral blood, including lymphocytes, monocytes, etc.

The term "T cell activation" or "T cell stimulation" and other grammatically forms thereof may refer to the state of T cells that are sufficiently stimulated to induce detectable cell proliferation, cytokine production, and/or detectable effector function. In some cases, "complete T cell activation" can be similar to the triggering of T cell cytotoxicity. Various assays known in the art can be used to determine T cell activation. The assay can be an ELISA to measure cytokine secretion, ELISPOT, a flow cytometry assay (CD107) for measuring intracellular cytokine expression, a flow cytometry assay for measuring proliferation, and a cytotoxicity assay (51Cr release assay) for determining target cell elimination. A control (non-engineered cell) is usually used in the assay to be compared with an engineered cell (CAR T) to determine the relative activation of the engineered cell compared to the control. In addition, the assay can be compared with engineered cells incubated or contacted with target cells that do not express the target antigen. For example, the comparison may be a comparison with GPC3-CART cells incubated with target cells that do not express GPC3.

When used to refer to a nucleotide sequence, the term "sequence" and other grammatical forms as used herein may include DNA or RNA, and may be single-stranded or double-stranded. The nucleic acid sequence can be mutated. The nucleic acid sequence can have any length.

The term "effective amount" as used herein refers to an amount that provides a therapeutic or prophylactic benefit.

The term "expression vector" as used herein refers to a vector comprising a recombinant polynucleotide, which comprises an expression regulatory sequence operatively linked to the nucleotide sequence to be expressed. The expression vector comprises sufficient cis-acting elements for expression; other elements for expression can be provided by host cells or in vitro expression systems. Expression vectors include all those known in the art, such as cosmids, plasmids (e.g. naked or contained in liposomes), and viruses (e.g., lentivirus, retrovirus, adenovirus, and adeno-associated virus).

The term "lentivirus" as used herein refers to a genus of the retroviridae family. Lentivirus is unique among retroviruses in their ability to infect non-dividing cells; they can deliver a large amount of genetic information into the DNA of host cells, so they are one of the most effective methods using gene delivery vehicles. HIV, SIV and FIV are all examples of lentiviruses. Vectors derived from lentiviruses provide a means to achieve significantly improved levels of gene transfer in vivo.

The term "vector" as used herein is a composition that contains an isolated nucleic acid and can be used to deliver the isolated nucleic acid into a cell. Many vectors are known in the art, including but not limited to linear polynucleotides, polynucleotides related to ionic or amphiphilic compounds, plasmids and viruses. Therefore, the term "vector" includes autonomously replicating plasmids or viruses. The term should also be interpreted to include non-plasmid and non-viral compounds that facilitate the transfer of nucleic acids into cells, such as polylysine compounds, liposomes, etc. Examples of viral vectors include, but are not limited to, adenovirus vectors, adeno-associated virus vectors, retroviral vectors, etc.

As used herein, the term sequence "identity" determines the percent identity by comparing two best-matched sequences over a comparison window (e.g., at least 20 positions), wherein the portion of the polynucleotide or polypeptide sequence in the comparison window may include additions or deletions (i.e. gaps), for example 20% or less gaps (e.g., 5 to 15%, or 10 to 12%) compared to the reference sequence (which does not contain additions or deletions) for the two sequences that best match. The percentage is usually calculated by determining the number of positions where the same nucleic acid base or amino acid residue occurs in the two sequences to obtain the number of correct matching positions. The number of correct matching positions is divided by the total number of positions in the reference sequence (i.e., the window size), and multiply the result by 100 to obtain the percentage of sequence identity.

The term "exogenous" as used herein refers to a nucleic acid molecule or polypeptide that has no endogenous expression in the cell, or the expression level is insufficient to achieve the function that it has when it is overexpressed. Thus, "exogenous" includes recombinant nucleic acid molecules or polypeptides expressed in cells, such as exogenous, heterologous and overexpressed nucleic acid molecules and polypeptides.

The term "endogenous" refers to a nucleic acid molecule or polypeptide derived from a gene in the organism's own genome. In some embodiments, the chimeric receptor of the invention is a chimeric antigen receptor. The term "Chimeric Antigen Receptor (CAR)" as used herein refers to a tumor antigen binding domain fused to an intracellular signaling domain that can activate T cells. Frequently, the extracellular binding domain of CAR is derived from mouse or humanized or human monoclonal antibodies.

Chimeric antigen receptors usually comprise (cell) extracellular antigen binding regions. In some embodiments, the extracellular antigen binding region may be fully human. In other cases, the extracellular antigen binding region can be humanized. In other cases, the extracellular antigen binding region may be of murine origin, or the chimera in the extracellular antigen binding region consists of amino acid sequences from at least two different animals. In some embodiments, the extracellular antigen binding region may be non-human. A variety of antigen binding regions can be designed. Non-limiting examples include single chain variable fragments (scFv) derived from antibodies, antigen binding regions of fragments (Fab) selected from libraries, single domain fragments, or natural ligands that bind to their homologous receptors. In some embodiments, the extracellular antigen binding region may comprise scFv, Fab, or natural ligands, and any derivatives thereof. The extracellular antigen binding region may refer to a molecule other than the intact antibody, which may comprise a part of the intact antibody and can bind to the antigen to which the intact antibody binds. Examples of antibody fragments may include, but are not limited to, Fv, Fab, Fab', Fab'-SH, F(ab')₂; bifunctional antibodies, linear antibodies; single-chain antibody molecules (such as scFv); and multispecific antibodies formed from antibody fragments. Extracellular antigen binding regions, for example scFv, Fab, or natural ligands, can be part of the CAR with determined antigen specificity. The extracellular antigen binding region can bind to any complementary target. The extracellular antigen binding region can be derived from antibodies with known variable region sequences. The extracellular antigen binding region can be obtained from antibody sequences obtained from available mouse hybridomas. Alternatively, the extracellular antigen binding region can be obtained from total extracellular cleavage sequencing of tumor cells or primary cells such as tumor infiltrating lymphocytes (TIL).

In some cases, the binding specificity of the extracellular antigen binding region can be determined by complementarity determining regions or CDRs, such as light chain CDRs or heavy chain CDRs. In many cases, the binding specificity can be determined by the light chain CDR and the heavy chain CDR. Compared with other reference antigens, the combination of a given heavy chain CDR and light chain CDR can provide a given binding pocket, which can confer greater affinity and/or specificity to the antigen (eg, GPC3). For example, glypican-3 specific CDRs can be expressed in the extracellular binding region of CARs, making GPC3-targeting CARs able to target T cells to GPC3-expressing tumor cells.

In certain aspects of any of the embodiments disclosed herein, the extracellular antigen binding region, such as a scFv, may comprise a light chain CDR specific for the antigen. The light chain CDR may be the complementarity determining region of the scFv light chain of an antigen binding unit such as a CAR. The light chain CDR may comprise a consecutive amino acid residue sequence, or two or more consecutive amino acid residue sequences separated by non-complementarity determining regions (e.g., a framework region). In some cases, a light chain CDR may comprise two or more light chain CDRs, which may be referred to as light chain CDR-1, CDR-2, etc. In some cases, a light chain CDR may comprise three light chain CDRs, which may be referred to as light chain CDR-1, light chain CDR-2, and light chain CDR-3, respectively. In some examples, a group of CDRs present on a common light chain can be collectively referred to as light chain CDRs.

In certain aspects of any of the embodiments disclosed herein, the extracellular antigen binding region, such as a scFv, may comprise a heavy chain CDR specific for an antigen. The heavy chain CDR may be the heavy chain complementarity determining region of an antigen binding unit such as a scFv. The heavy chain CDR may comprise a consecutive amino acid residue sequence, or two or more consecutive amino acid residue sequences separated by non-complementarity determining regions (such as a framework region). In some cases, a heavy chain CDR may include two or more heavy chain CDRs, which may be referred to as heavy chain CDR-1, CDR-2, etc. In some cases, the heavy chain CDR may include three heavy chain CDRs, which may be referred to as heavy chain CDR-1, heavy chain CDR-2, and heavy chain CDR-3, respectively. In some cases, a group of CDRs present on a common heavy chain can be collectively referred to as heavy chain CDRs.

By using genetic engineering, extracellular antigen binding regions can be modified in various ways. In some cases, extracellular antigen binding regions can be mutated so that the extracellular antigen binding regions can be selected to have a higher affinity for their targets. In some cases, the affinity of an extracellular antigen binding region for its target can be optimized for targets that are expressed at low levels on normal tissues. This optimization can be done to minimize potential toxicity. In other cases, clones of extracellular antigen-binding regions may have higher affinity for the membrane-bound forms of a target rather than the soluble form counterparts. This kind of modifications can be made because different levels of targets in soluble form can also be detected, and their being targeted can cause undesirable toxicity.

In some cases, the extracellular antigen binding region comprises a hinge or spacer. The terms hinge and spacer can be used interchangeably. The hinge can be considered as part of the CAR, used for providing flexibility to the extracellular antigen binding region. In some cases, the hinge can be used to detect the CAR on the cell surface, especially when the antibody that detects the extracellular antigen binding region is ineffective or available. For example, the length of the hinge derived from immunoglobulin may require optimization, depending on the location of the epitope on the target targeted by the extracellular antigen binding region.

In some cases, the hinge may not belong to an immunoglobulin, but belong to another molecule, such as the natural hinge of a CD8α molecule. The CD8α hinge may contain cysteine and proline residues that are known to play a role in the interaction of CD8 co-receptors and MHC molecules. The cysteine and proline residues can affect the performance of the CAR. The size of CAR hinge can be adjusted. This morphology of the immune synapse between T cells and target cells also limits the distance that cannot be functionally bridged by CAR due to the distal membrane epitopes on cell surface target molecules, i.e. using CAR with short hinge also cannot make the synapse distance to reach the approximate value that the signal can conduct. Similarly, signal output of proximal membrane epitope in CAR-targeting antigen is only observed in the context of a long hinge CAR. The hinge can be adjusted according to the extracellular antigen binding region used. The hinge can be of any length. A transmembrane domain can anchor the CAR to the plasma membrane of a cell. The natural transmembrane portion of CD28 can be used in CAR. In other cases, the natural transmembrane portion of CD8α can also be used in CAR. "CD8" can be a protein that has at least 85, 90, 95, 96, 97, 98, 99, or 100% identity with NCBI reference number: NP_001759 or a fragment thereof having stimulating activity. The "CD8 nucleic acid molecule" can be a polynucleotide encoding a CD8 polypeptide. In some cases, the transmembrane region can be the natural transmembrane portion of CD28. "CD28" can refer to a protein having at least 85, 90, 95, 96, 97, 98, 99, or 100% identity with NCBI reference number: NP_006130 or a fragment thereof having stimulating activity. The "CD28 nucleic acid molecule" may be a polynucleotide encoding a CD28 polypeptide. In some cases, the transmembrane portion may comprise the CD8α region. The intracellular signaling domain of CAR may be responsible for activating at least one of the effector functions of T cells in which the CAR has been placed. CAR can induce effector functions of T cells, for example, the effector function is cytolytic activity or helper activity, including cytokine secretion. Therefore, the term "intracellular signaling domain" refers to the part of a protein that transduces effector function signals and guides cells to perform specific functions. Although the entire intracellular signaling region can usually be used, in many cases it is not necessary to use the entire chain of a signaling domain. In some cases, truncated portions of intracellular signaling regions are used. In some cases, the term intracellular signaling domain is therefore intended to include any truncated portion of the intracellular signaling region sufficient to transduce effector function signals.

Preferred examples of signaling domains used in CAR may include T cell receptor (TCR) cytoplasmic sequences and co-receptors that act synergistically to initiate signaling after target-receptor binding, as well as any derivatives or variant sequence thereof and any synthetic sequence with the same functionality of these sequences.

In some cases, the intracellular signaling domain may contain a known immunoreceptor tyrosine activation motif (ITAM) signaling motif. Examples of ITAMs containing cytoplasmic signaling sequences include functional signaling domains derived from proteins of TCRζ, FcRγ, FcRβ, CD3γ, CD3δ, CD3ε, CD5, CD22, CD79a, CD79b, DAP10 of CD66d, or DAP12. However, in a preferred embodiment, the intracellular signaling domain is derived from the CD3ζ chain. An example of a T cell signaling domain containing one or more ITAM motifs is the CD3ζ domain, also known as the T cell receptor T3ζ chain or CD247. This domain is part of the T cell receptor-CD3 complex, and plays an important role in combining the antigen recognition of several intracellular signaling pathways with the main effect activation of T cells. As used herein, CD3ζ mainly refers to human CD3ζ and its isoforms, as known from Swissprot entry P20963, including proteins with substantially the same sequence. As part of the chimeric antigen receptor, once again, the whole T cell receptor T3ζ chain is not required, and any derivative containing the signaling domain of the T cell receptor T3ζ chain is suitable, including any functional equivalents thereof .

The intracellular signaling domain can be selected from any one of the domains in Table 1. In some cases, the domain can be modified so that the identity with the reference domain can be about 50% to about 100%. Any one of the domains of Table 1 can be modified so that the modified form can contain about 50, 60, 70, 80, 90, 95, 96, 97, 98, 99 or at most about 100% identity. The intracellular signaling region of the CAR may further include one or more costimulatory domains. The intracellular signaling region may contain a single costimulatory domain, such as the ζ chain (first-generation CAR) or with CD28 or 4-1BB (second-generation CAR). In other examples, the intracellular signaling region may contain two costimulatory domains, such as CD28/OX40 or CD28/4-1BB (third generation).

Together with intracellular signaling domains such as CD8, these costimulatory domains can produce downstream activation of the kinase pathway, thereby supporting gene transcription and functional cellular responses. The costimulatory domain of CAR can activate CD28 (phosphatidylinositol-4,5-bisphosphate 3-kinase) or 4-1BB/OX40 (TNF-receptor-related factor adaptor protein) pathways, as well as MAPK and Akt activation related proximal signal proteins.

In some cases, the signal generated by the CAR may be combined with auxiliary or costimulatory signals. For costimulatory signaling domains, the chimeric antigen receptor-like complex can be designed to contain several possible costimulatory signaling domains. As is well known in the art, in naive T cells, T cell receptor engagement alone is not sufficient to induce the complete activation of T cells into cytotoxic T cells. The activation of intact productive T cells requires a second costimulatory signal. Several receptors that provide costimulation for T cell activation have been reported, including but not limited to CD28, OX40, CD27, CD2, CD5, ICAM-1, LFA-1 (CD11a/CD18), 4-1BBL, MyD88 and 4- 1BB. The signaling pathways used by these costimulatory molecules can all act synergistically with the primary T cell receptor activation signal. The signals provided by these costimulatory signaling regions can cooperate with the primary effect activation signals derived from one or more ITAM motifs (such as the CD3 zeta signaling domain), and can complete the requirement of T cell activation.

In some cases, adding costimulatory domains to chimeric antigen receptor-like complexes can enhance the efficacy and durability of engineered cells. In another embodiment, T cell signaling domains and costimulatory domains are fused to each other to form a signaling region.

**Table 1. Costimulatory domain**

| Gene marks | Abbreviations | Names |
|---|---|---|
| CD27 | CD27; T14; S152; Tp55; TNFRSF7; S152. LPFS2 | CD27 molecule |
| CD28 | Tp44; CD28; CD28 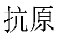 | CD28 molecule |
| TNFRSF9 | ILA; 4-1BB; CD137; CDw137 | tumor necrosis factor receptor family member 9 |
| TNFRSF4 | OX40; ACT35; CD134; IMD16; TXGP1L | tumor necrosis factor receptor family member 4 |
| TNFRSF8 | CD30; Ki-1; D1S166E | tumor necrosis factor receptor family member 8 |
| CD40LG | IGM; IMD3; TRAP; gp39; CD154; CD40L; HIGM1;T-BAM; TNFSF5; hCD40L | CD40 ligand |
| ICOS | AILIM; CD278; CVID1 | Inducible T cell costimulator |
| ITGB2 | LAD; CD18; MF17; MFI7; LCAMB; LFA-1; MAC-1 | Integrin β2 (complement component 3 receptor 3 and 4 subunit) |
| CD2 | T11; SRBC; LFA-2 | CD2 molecule |
| CD7 | GP40; TP41; Tp40; LEU-9 | CD7 molecule |
| KLRC2 | NKG2C; CD159c; NKG2-C | Killer cell lectin-like receptor subfamily C, member 2 |
| TNFRSF18 | AITR; GITR; CD357; GITR-D | tumor necrosis factor receptor family member 18 |
| TNFRSF14 | TR2; ATAR; HVEA; HVEM; CD270; LIGHTR | tumor necrosis factor receptor family member 14 |
| HAVCR1 | TIM; KIM1; TIM1; CD365; HAVCR; KIM-1; TIM-1;TIMD1; TIMD-1; HAVCR-1 | Hepatitis A Virus Cell Receptor 1 |
| LGALS9 | HUAT; LGALS9A, Galectin-9 | Lectin, galactoside binding, soluble, 9 |
| CD83 | BL11; HB15 | CD83 molecule |

The term "regulating" as used herein refers to a positive or negative change. Examples of regulating include 1%, 2%, 10%, 25%, 50%, 75%, or 100% changes.

The term "treatment" as used herein refers to clinical intervention in the process of trying to change an individual or treating a disease caused by cells. It can be used for prevention or intervention in the clinical pathological process. The therapeutic effects include, but are not limited to, preventing the occurrence or recurrence of the disease, reducing symptoms, reducing the direct or indirect pathological consequences of any disease, preventing metastasis, slowing down the progression of the disease, improving or relieving the condition, alleviating or improving the prognosis, etc.

### T cell

The T cell described herein refers to a T cell modified by the method of the present invention, and the endogenous TCR genes and/or MHC genes of the T cell are silenced.

In some cases, the T cells may be stem memory TSCM cells composed of CD45RO(-), CCR7(+), CD45RA(+), CD62L+(L-selectin), CD27+, CD28+ and/or IL-7Rα+. The stem memory cells can also express CD95, IL-2Rβ, CXCR3 and/or LFA-1, and show many functional properties that are different from the stem memory cells. Alternatively, immunoreactive cells may also be a central memory TCM cell containing L-selectin and CCR7, wherein the central memory cell can secrete, for example IL-2, but not IPNγ or IL-4. The immunoreactive cells can also be effector memory TEM cells containing L-selectin or CCR7, and produce, for example, effector cytokines such as IPNγ and IL-4.

The delivery of vectors is usually by systemic administering (e.g., intravenous, intraperitoneal, intramuscular, subcutaneous or intracranial infusion) or topical application to individual patients in vivo, as described below. Alternatively, vectors can be delivered to cells ex vivo, for example, cells removed from an individual patient (e.g., lymphocytes, T cells, bone marrow aspirate, tissue biopsy), and then the cells are usually re-implanted into the patient's body after the selection for those incorporated with vectors. Before or after the selection, the cells can be expanded.

The T cells can be obtained from many sources, including PBMCs, bone marrow, lymph node tissues, umbilical cord blood, thymus tissues, and tissues from infection sites, ascites, pleural effusion, spleen tissues, and tumor tissues. In some cases, any number of techniques known to those skilled in the art, such as Ficoll™ isolation, can be used to obtain T cells from blood collected from an individual. In one embodiment, cells from the circulating blood of the individual are obtained by apheresis. Apheresis products usually comprise lymphocytes, including T cells, monocytes, granulocytes, B cells, other nucleated white blood cells, red blood cells and platelets. In one embodiment, the cells collected by apheresis collection can be washed to remove the plasma fraction and placed in a suitable buffer or medium for subsequent processing steps. Alternatively, cells can be derived from healthy donors, from patients diagnosed with cancer.

In some embodiments, the cells may be part of a mixed cell population with different phenotypic characteristics. Cell lines from transformed T cells according to the aforementioned method can also be obtained. The cells can also be obtained from cell therapy banks.

In some cases, suitable primary cells include peripheral blood mononuclear cells (PBMC), peripheral blood lymphocytes (PBL) and other blood cell subpopulations, such as but not limited to T cells, natural killer cells, monocytes, natural Killer T cells, monocyte precursor cells, hematopoietic stem cells or non-pluripotent stem cells. In some cases, the cell may be any T cell such as tumor infiltrating cells (TIL), such as CD3+ T cells, CD4+ T cells, CD8+ T cells, or any other type of T cells. T cells may also include memory T cells, memory stem T cells or effector T cells. T cells can also be selected from a great number of populations, for example from whole blood. T cells can also be expanded from a great number of populations. T cells may also tend to a specific population and phenotype. For example, T cells can be tend to phenotypes including CD45RO(-), CCR7(+), CD45RA(+), CD62L(+), CD27(+), CD28(+), and/or IL-7Ra(+). Suitable cells can have one or more markers selected from the group consisting of that in the following list: CD45RO(-), CCR7(+), CD45RA(+), CD62L(+), CD27(+), CD28(+) and/or IL-7Ra(+). Suitable cells also include stem cells, for example, embryonic stem cells, induced pluripotent stem cells, hematopoietic stem cells, neuronal stem cells, and mesenchymal stem cells. Suitable cells may include any number of primary cells, such as human cells, non-human cells, and/or mouse cells. Suitable cells may be progenitor cells. Suitable cells can be derived from the subject to be treated (e.g., patient).

The amount of therapeutically effective cells required in a patient can vary depending on the viability of the cells and the efficiency with which the cells are genetically modified (for example, the efficiency with which the transgene is integrated into one or more cells, or the expression level of the protein encoded by the transgene). In some cases, the cell viability result after genetic modification (e.g., doubling) and the efficiency of transgene integration may correspond to the therapeutic amount of cells that can be used for administration to the subject. In some cases, the increase in cell viability after genetic modification may correspond to a decrease in the amount of required cells that are effective for the patient in the treatment. In some cases, an increase in the efficiency of integration of the transgene into one or more cells may correspond to a decrease in the amount of required cells that are therapeutically effective for the patient. In some cases, determining the required therapeutically effective amount of cells can comprise determining functions related to changes in the cells over time. In some cases, determining the required therapeutically effective amount of cells may comprise determining the function (e.g., cell culture time, electrotransfection time, cell culture time, electrotransfection time, cell Stimulation time) related to the efficiency changes of transgene integration into one or more cells. In some cases, the therapeutically effective cell may be a cell population that comprises about 30% to about 100% expression of chimeric receptors on the cell surface. In some cases, as measured by flow cytometry, therapeutically effective cells can express the chimeric receptor on the surface of about 30%, 35%, 40%, 45%, 50%, 55%, 60%, 65%, 70%, 75% 80%, 85%, 90%, 91%, 92%, 93%, 94%, 95%, 96%, 97%, 98%, 99%, 99.5%, 99.9% or more than about 99.9% of the cells.

### Pharmaceutical composition

The T cell of the present invention can be used to prepare pharmaceutical compositions. In addition to the effective amount of the T cells, the pharmaceutical composition may also comprise pharmaceutically acceptable carriers. The term "pharmaceutically acceptable" means that when the molecular entities and compositions are properly administered to animals or humans, they will not produce adverse, allergic or other adverse reactions.

Some specific examples of substances that can be used as pharmaceutically acceptable carriers or components thereof are antioxidants; preservatives; pyrogen-free water; isotonic salt solutions; and phosphate buffers etc.

The composition of the present invention can be prepared into various dosage forms according to needs, and doctors can determine the beneficial dosage for a patient according to factors such as the patient's type, age, weight, general disease condition, and administration method. The method of administration can be, for example, parenteral administration (such as injection) or other treatment methods.

"Parenteral" administration of the composition includes, for example, subcutaneous (s.c.), intravenous (i.v.), intramuscular (i.m.) or intrasternal injection or infusion techniques.

The T cell population-containing preparation administered to an individual comprises multiple T cells effective in treating and/or preventing a specific indication or disease. Therefore, a therapeutically effective population of immunoreactive cells can be administered to an individual. Generally, a preparation comprising about 1×10⁴ to about 1×10¹⁰ immunoreactive cells is administered. In most cases, the preparation will contain about 1×10⁵ to about 1×10⁹ immunoreactive cells, about 5×10⁵ to about 5×10⁸ immunoreactive cells, or about 1×10⁶ to about 1×10⁷ immunoreactive cells. However, depending on the location, source, identity, degree and severity of the cancer, the age and physical condition of the individual to be treated, etc., the amount of CAR immunoreactive cells administered to an individual will vary within a wide range. The doctor will finally determine the appropriate dose to be used.

In some embodiments, chimeric antigen receptors are used to stimulate immune cell-mediated immune responses. For example, a T cell-mediated immune response is an immune response involving T cell activation. Activated antigen-specific cytotoxic T cells can induce apoptosis in target cells displaying foreign antigen epitopes on the surface, such as cancer cells displaying tumor antigens. In another embodiment, chimeric antigen receptors are used to provide anti-tumor immunity in mammals. Due to the T cell-mediated immune response, the subject will develop anti-tumor immunity.

In some cases, the method for treating a subject with cancer may involve the administration of one or more T cells of the present invention to the subject in need of treatment. The T cells can bind tumor target molecules and induce the death of cancer cells.

As described above, the present invention also provides a method for treating pathogen infection in an individual, which comprises administering to the individual a therapeutically effective amount of the T cells of the present invention.

### Combination with anti-tumor drugs

In some embodiments, the T cells of the present invention can be administered in combination with another therapeutic agent. In some embodiments, the other therapeutic agent is a chemotherapeutic drug. The chemotherapeutic drugs that can be used in combination with the T cells of the present invention include, but are not limited to, mitotic inhibitors (vinca alkaloids), including vincristine, vinblastine, vindesine, and novibin (TM) (vinorelbine, 5'-dehydrohydrogen sulfide); topoisomerase I inhibitors, for example camptothecin compounds, including Camptosar™ (irinotecan HCL), Hycamtin™ (topotecan HCL) and other compounds derived from camptothecin and its analogues; podophyllotoxin derivatives, for example etoposide, teniposide and mitopodozide( 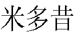 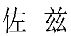 ); alkylating agents cisplatin, cyclophosphamide, nitrogen mustard, trimethylene thioxophosphamide, carmustine, busulfan, chlorambucil, belustine( 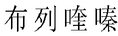 ), uracil mustard, chlomaphazine( 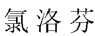 ) and dacarbazine; antimetabolites, including cytarabine, fluorouracil, methotrexate, mercaptopurine, azathioprine and procarbazine; antibiotics, including but not limited to doxorubicin, bleomycin, dactinomycin, daunorubicin, mithramycin( 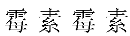 ), mitomycin, sarcomycin C, and daunorubicin; and other chemotherapeutic drugs, including but not limited to anti-tumor antibodies, dacarbazine, azacytidine, amsacrine( 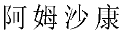 ), melphalan, ifosfamide and mitoxantrone.

In some embodiments, the chemotherapeutic drugs that can be used in combination with the T cells of the present invention include, but are not limited to, anti-angiogenic agents, including anti-VEGF antibodies (including humanized and chimeric antibodies, anti-VEGF aptamers, and antisense oligonucleotides). and other angiogenesis inhibitors, such as angiostatin, endostatin, interferon, retinoic acid, and tissue inhibitors of metalloproteinase-1 and -2.

### Kit

The present invention also provides a kit comprising the T cell of the present invention. The kit can be used to treat or prevent cancer, pathogen infection, immune disorder, or allogeneic transplantation. In one embodiment, the kit may comprise a therapeutic or prophylactic composition which comprises an effective amount of T cells in one or more unit dosage forms.

In some embodiments, the kit comprises a sterile container that can contain a therapeutic or prophylactic composition.

In some cases, the kit may comprise about 1×10⁴ cells to about 1×10⁶ cells. In some cases, the kit may comprise at least about 1×10⁵ cells, at least about 1×10⁶ cells, at least about 1×10⁷ cells, at least about 4×10⁷ cells, at least about 5×10⁷ cells, at least about 6×10⁷ cells, at least about 6×10⁷ cells, 8×10⁷ cells, at least about 9×10⁷ cells, at least about 1×10⁸ cells, at least about 2×10⁸ cells, at least about 3×10⁸ Cells, at least about 4×10⁸ cells, at least about 5×10⁸ cells, at least about 6×10⁸ cells, at least about 6×10⁸ cells, at least about 8×10⁸ cells, at least about 9×10⁸ cells, at least about 1×10⁹ cells, at least about 2×10⁹ cells, at least about 3×10⁹ cells, at least about 4×10⁹ cells, at least about 5×10⁹ cells, at least about 6×10⁹ cells, at least about 8×10⁹ cells, at least about 9×10⁹ cells, at least about 1×10¹⁰ cells, at least about 2×10¹⁰ cells, at least about 3×10¹⁰ cells, at least about 4×10¹⁰ cells, at least about 5×10¹⁰ cells, at least about 6×10¹⁰ cells, at least about 9×10¹⁰ cells, at least about 9×10¹⁰ cells, at least about 1×10¹¹ cells, at least about 2×10¹¹ cells, at least about 3×10¹¹ cells, At least about 4×10¹¹ cells, at least about 5×10¹¹ cells, at least about 8×10¹¹ cells, at least about 9×10¹¹ cells, or at least about 1×10¹² cells. For example, about 5×10¹⁰ cells can be comprised in the kit.

In some cases, the kit may comprise allogeneic cells. In some cases, the kit can comprise cells that can comprise genomic modifications. In some cases, the kit may comprise "off-the-shelf" cells. In some cases, the kit can comprise cells that can be expanded for clinical use. In some cases, the kit may comprise contents for research purposes.

### The advantages of the present invention:

Gene editing according to the method of the present invention not only has high editing efficiency, but also has a great cell viability.

The present invention will be further explained below in conjunction with specific examples. It should be understood that these examples are only used to illustrate the present invention and not to limit the scope of the present invention. Experimental methods without specific conditions in the following examples usually follows the conventional conditions as described in J. Sambrook et al., Molecular Cloning Experiment Guide, Third Edition, Science Press, 2002, or according to the conditions suggested by the manufacturer.

Exemplary, in the following examples, T cells are selected to illustrate the method of the present invention.

The obtaining of T cells: human peripheral blood mononuclear cells (PBMCs) are isolated from peripheral blood collected from healthy donors, activated by adding CD3/CD28 antibody-conjugated beads, and then cultured and expanded to obtain T cells.

### EXAMPLE

### Example 1. Design and synthesis of sgRNA targeting TRAC gene

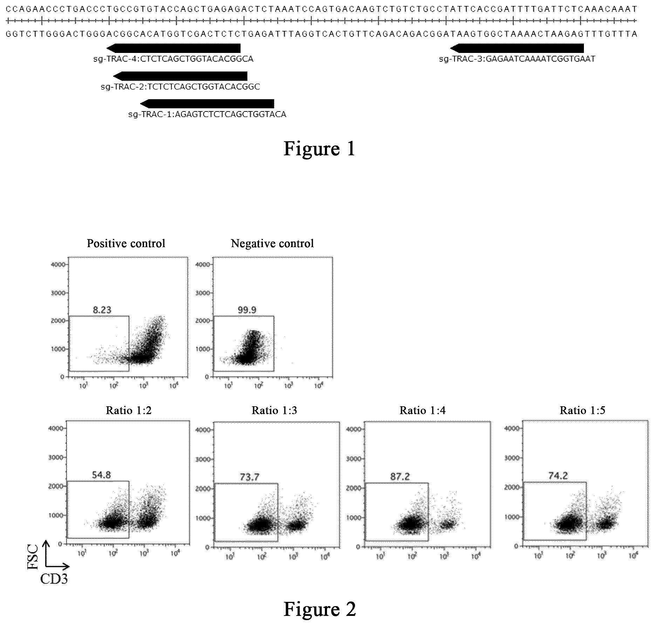

Aiming at the first exon of the TRAC (TCRaC, constant locus of T cell receptor α) gene (the nucleotide sequence is shown in SEQ ID NO:1)(as shown in Figure 1), eight sgRNA sequences targeting the TRAC gene sg-TRAC-1 (SEQ ID NO: 2), sg-TRAC-2 (SEQ ID NO: 3), sg-TRAC-3 (SEQ ID NO: 4), sg-TRAC-4 (SEQ ID NO: 4), sg-TRAC-5 (SEQ ID NO: 32), sg-TRAC-6 (SEQ ID NO: 33), sg-TRAC-7 (SEQ ID NO: 39), and sg-TRAC-8 (SEQ ID NO: 40) are designed and obtained.

Sg-TRAC-1 (SEQ ID NO: 2), sg-TRAC-2 (SEQ ID NO: 3), sg-TRAC-3 (SEQ ID NO: 4), sg-TRAC-5 (SEQ ID NO: 32), sg-TRAC-6 (SEQ ID NO: 33), sg-TRAC-7 (SEQ ID NO: 39) and sg-TRAC-8 (SEQ ID NO: 40) are selected for the test. Primers shown in SEQ ID NOs: 20 and 21 are synthesized *in vitro, in vitro* gRNA transcription kit is purchased from Thermo Fisher, and sg-TRAC-1 is transcribed and amplified. Primers shown in SEQ ID NOs: 22 and 23 are synthesized *in vitro, in vitro* gRNA transcription kit is purchased from Thermo Fisher, and sg-TRAC-2 is transcribed and amplified. Primers shown in SEQ ID NOs: 24 and 25 are synthesed *in vitro, in vitro* gRNA transcription kit is purchased from Thermo Fisher, and sg-TRAC-3 is transcribed and amplified. Primers shown in SEQ ID NOs: 34 and 35 are synthesized *in vitro, in vitro* gRNA transcription kit is purchased from Thermo Fisher, and sg-TRAC-5 is transcribed and amplified. Primers shown in SEQ ID NOs: 36 and 37 are synthesized *in vitro, in vitro* gRNA transcription kit is purchased from Thermo Fisher, and sg-TRAC-6 is transcribed and amplified. Primers shown in SEQ ID NOs: 41 and 42 are synthesized *in vitro, in vitro* gRNA transcription kit is purchased from Thermo Fisher, and sg-TRAC-7 is transcribed and amplified. Pimers shown in SEQ ID NOs: 43 and 44 are synthesized *in vitro, in vitro* gRNA transcription kit is purchased from Thermo Fisher, and sg-TRAC-8 is transcribed and amplified.
TRAC-exon 1 sequence (SEQ ID NO:1): AACGCCTTCAACAACAGCATTATTCCAGAAGACACCTTCTTCCCCAGCCCAGG 
ATATCCAGAACCCTGACCCTGCCGTGTACCAGCTGAGAGACTCTAAATCCAGTGA
CAAGTCTGTCTGCCTATTCACCGATTTTGATTCTCAAACAAATGTGTCACAAAGTA
AGGATTCTGATGTGTATATCACAGACAAAACTGTGCTAGACATGAGGTCTATGGA
CTTCAAGAGCAACAGTGCTGTGGCCTGGAGCAACAAATCTGACTTTGCATGTGCA
sg-TRAC-1 (SEQ ID NO: 2): AGAGTCTCTCAGCTGGTACA
sg-TRAC-2 (SEQ ID NO: 3): TCTCTCAGCTGGTACACGGC
sg-TRAC-3 (SEQ ID NO: 4): GAGAATCAAAATCGGTGAAT
sg-TRAC-4 (SEQ ID NO: 5): CTCTCAGCTGGTACACGGCA
sg-TRAC-5 (SEQ ID NO: 32): GTCTCTCAGCTGGTACA
sg-TRAC-6 (SEQ ID NO: 33): AGTCTCTCAGCTGGTACA
sg-TRAC-7 (SEQ ID NO: 39): TTAGAGTCTCTCAGCTGGTACA
sg-TRAC-8 (SEQ ID NO: 40): TTTAGAGTCTCTCAGCTGGTACA

### Example 2. The effect of different ratios of Cas 9 enzyme and sg-TRAC on knockout efficiency

Activated T cells are taken for cell count and adjusted to a cell density of 2*10^7/ml. Sg-TRAC-1 (SEQ ID NO: 2) is selected as sgRNA.

The Cas 9 enzyme (purchased from NEB) and the sg-TRAC-1 are mixed in a molar ratios of 1:2, 1:3, 1:4, and 1:5 to form an RNP complex. After incubating for 10 minutes at room temperature, they are added to 1*10^6 T cells (the final concentration of the Cas 9 enzyme is 0.3µM). Wherein, the number of moles of the sg-TRAC-1 is calculated based on the base composition of the gRNA and a concentration of 4.03µg/µl (OD260/OD280=1.98).

The RNP complex is introduced into T cells using a BTX electrotransfection instrument (Harvard Apparatus, USA), and the electrotransfection parameters are 250V, 5ms. On the 5th day after transfection, T cells are taken for CD3 antibody (BD Biosciences) staining for flow cytometry to verify the efficiency of TCR knockout. The results of flow cytometry are shown in Figure 2 and Table 1: when the molar ratio of the Cas 9 enzyme to the sg-TRAC-1 is 1:3∼1:5, the knockout efficiency is above 70%. When the molar ratio of the Cas 9 enzyme to the sg-TRAC-1 is 1:4, the knockout efficiency is the highest, reaching 87.2%. It shows that when the molar ratio of the Cas 9 enzyme and the gRNA is 1:4, it has the best gene knockout efficiency.

**Table 1. Statistics of TCR knockout results using RNPs with different composition ratios**

| RNP ratio (0.3µM Cas9) | 1:2 | 1:3 | 1:4 | 1:5 |
|---|---|---|---|---|
| KO efficency (Day 5) | 54.8% | 73.7% | 87.2% | 74.2% |

### Example 3. Effects of different sgRNAs on TRAC gene knockout

3 different sgRNAs targeting the TRAC gene are selected from: sg-TRAC-1, sg-TRAC-2, sg-TRAC-3.

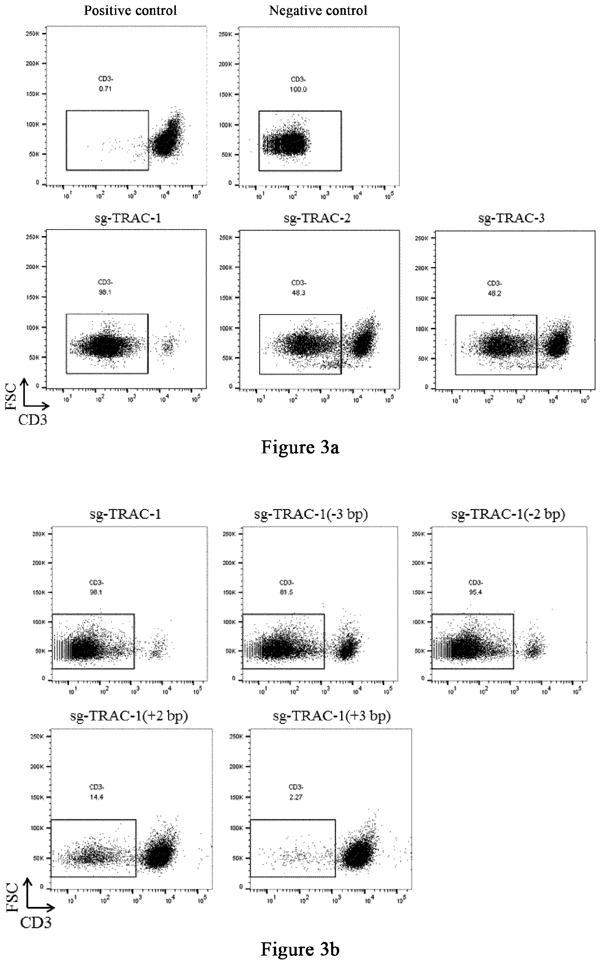

The effect of TRAC gene knockout is detected. The three sgRNAs in Example 1, sg-TRAC-1, sg-TRAC-2, and sg-TRAC-3 are synthesized and separately mixed with the Cas9 enzymes (0.5µM) at a ratio of 4:1 to form an RNP complex. Electrotransfection is performed to introduce the RNA complex into T cells, using a Maxcyte electrotransfection instrument (Maxcyte, Inc.) and based on the set parameters in the instrument. On the 5th day after transfection, T cells are taken for CD3 antibody (BD Biosciences) staining for flow cytometry to verify the efficiency of TCR knockout. The results of flow cytometry are shown in Figure 3 and Table 2. The knockout effect of the sg-TRAC-1 is significantly superior to that of the sg-TRAC-2 and sg-TRAC-3, indicating that the sg-TRAC-1 has the best knockout effect. At the same time, the effects of different lengths of the sg-TRAC-1 on the knockout efficiency are detected. Four sgRNAs (g-TRAC-1(-3 bp) (sg-TRAC-5), sg-TRAC-1(-2 bp) (sg-TRAC-6), sg-TRAC-1(+3bp) (sg-TRAC-7), sg-TRAC-1(+2 bp) (sg-TRAC-8)) are synthesized, and respectively mixed with Cas9 enzymes (0.5µM) with the molar ratio of 4:1 to form the RNP complex, which is introduced into T cells under the above conditions. On the 5th day after transfection, T cells are taken for CD3 antibody staining for flow cytometry to verify the knockout efficiency of TCR. The experimental results are shown in Figure 3b. Truncating the sg-TRAC-1 by 2 or 3 bases has little effect on the TCR knockout efficiency, while adding 2 or 3 bases will reduce the TCR knockout efficiency. It shows that the length of the sgRNA designed for this site can be changed to a certain extent, especially truncating it within 3 bases can also achieve a relatively high knockout effect.

**Table 2. Statistics of TCR knockout results using different gRNA sequences**

| sgRNA | sg-TRAC-1 | sg-TRAC-2 | sg-TRAC-3 |
|---|---|---|---|
| KO efficency (Day 5) | 98.1% | 48.3% | 48.2% |

It should be pointed out that although in the published article, sg-TRAC-1 could also make the TCR knockout efficiency reach 90%, the article adopted an optimized knockout method with two electrotransfections, and the process is relatively complex (refer to Clin Cancer Res. 2017 May 1;23(9):2255-2266, Fig. 1A showing that the TCR knockout rate is 95.7%). While we can achieve a knockout rate of more than 90% with a single electrotransfection, which has obvious advantages.

### Example 4: The effect of Cas 9 enzyme concentration on TRAC gene knockout

Sg-TRAC-1 (SEQ ID NO: 2) is selected as sgRNA, and when the molar ratio of Cas 9 enzymes and sg-TRAC-1 is 1:4, different concentrations of the Cas 9 enzymes (0.0625 µM, 0.125µM, 0.25µM, 0.5µM) are set to be detected for their effects on TRAC gene knockout.

After an RNP complex is incubated at room temperature for 10 minutes, the Maxcyte electrotransfection instrument (Maxcyte, Inc.) is used to introduce the RNP complex into T cells based on the set conditions of the instrument,. On the 5th day after transfection, T cells are taken for CD3 antibody (BD Biosciences) staining for flow cytometry to verify the efficiency of the TCR knockout.

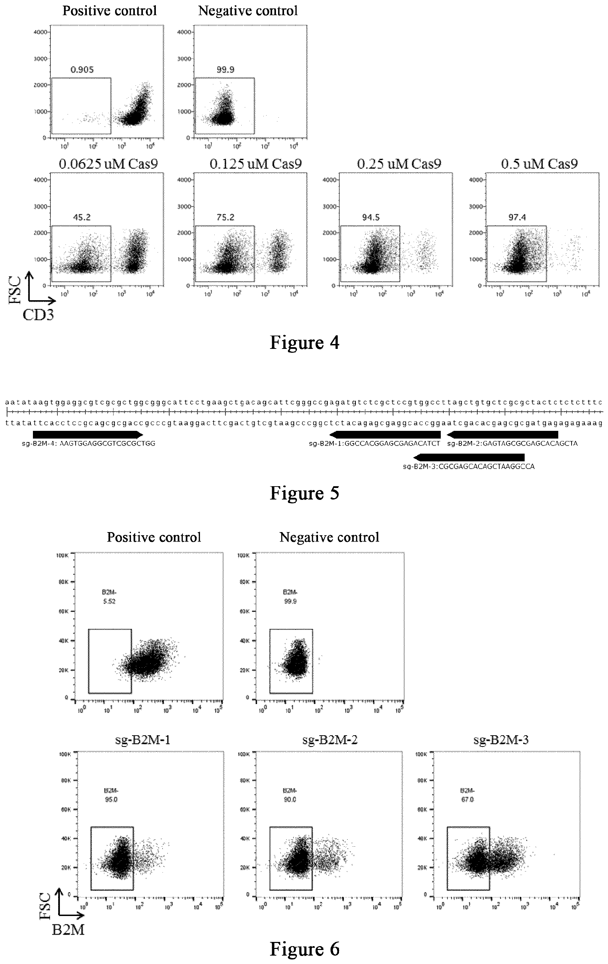

The results of flow cytometry are shown in Figure 4 and Table 3. When the concentration of the Cas9 enzymes is greater than 0.1µM, the knockout efficiency of TCR could reach more than 70%. For example, at the concentration of 0.125µM, the knockout efficiency of TCR could reach more than 75%; especially when the concentration is greater than 0.2µM, the knockout efficiency of TCR can reach more than 90%, such as at the concentration of 0.25µM, the knockout efficiency of TCR could reach more than 94.5%; when the concentration of the Cas9 enzymes is 0.3-0.5µM, it can reach more than 95%. When the concentration of the Cas9 enzymes is 0.5µM, the knockout efficiency of TCR can reach 97.4%, and the cell viability is more than 90%.

**Table 3. Statistics of TCR knockout results using different concentrations of the Cas9 enzymes**

| Cas9 enzyme concentration (RNP ratio 1: 4) | 0.0625 µM | 0.125µM | 0.25µM | 0.5µM |
|---|---|---|---|---|
| KO efficency (Day 5) | 45.2% | 75.2% | 94.5% | 97.4% |
| Cell viability (24 h) | 93% | 93% | 94% | 91% |

### Example 5 Design and synthesis of B2M gene-targeting sgRNAs

As shown in Figure 5, according to the first exon of the B2M gene, B2M-exon 1 (the nucleotide sequence is shown in SEQ ID NO: 10), four sgRNA sequences targeting the B2M gene (sg-B2M-1 (SEQ ID NO : 11), sg-B2M-2 (SEQ ID NO: 12), sg-B2M-3 (SEQ ID NO: 13), sg-B2M-4 (SEQ ID NO: 14)) are obtained.

Sg-B2M-1, sg-B2M-2, sg-B2M-3 are selected for the test. Primers shown in SEQ ID NOs: 26 and 27 are synthesized *in vitro, in vitro* gRNA transcription kit is purchased from Thermo Fisher, and the sg-B2M-1 is transcribed and amplified. Primers shown in SEQ ID NOs: 28 and 29 are synthesized *in vitro, in vitro* gRNA transcription kit is purchased from Thermo Fisher, and the sg-B2M-2 is transcribed and amplified. Primers shown in SEQ ID NOs: 30 and 31 are synthesized *in vitro, in vitro* gRNA transcription kit is purchased from Thermo Fisher, and the sg-B2M-3 is transcribed and amplified.
B2M-exon 1 sequence (SEQ ID NO: 10): 
AATATAAGTGGAGGCGTCGCGCTGGCGGGCATTCCTGAAGCTGACAGCATTCGGGCCGAG
ATGTCTCGCTCCGTGGCCTTAGCTGTGCTCGCGCTACTCTCTCTTTCTGGCCTGGAGGCTAT
CCAGC
sg-B2M-1 (SEQ ID NO: 11): GGCCACGGAGCGAGACATCT
sg-B2M-2 (SEQ ID NO: 12): GAGTAGCGCGAGCACAGCTA
sg-B2M-3 (SEQ ID NO: 13): CGCGAGCACAGCTAAGGCCA

### Example 6. Effects of different sgRNA sequences on B2M gene knockout.

With regard to the B2M gene-targeting sgRNA sequences, sg-B2M-1, sg-B2M-2, and sg-B2M-3, obtained in Example 5, the effects on B2M gene knockout are compared.

After mixing Cas9 enzymes (0.5µM) and gRNAs at a molar ratio of 1:4 to form an RNP complex, the Maxcyte electrotransfection instrument (Maxcyte, Inc.) is used to introduce the RNP complex into T cells based on the set conditions of the instrument. On the 5th day after transfection, T cells are taken staining with β-microglobulin antibody (BD Biosciences) for flow cytometry to verify the efficiency of B2M knockout. The flow cytometry results are shown in Figure 6 and Table 4. The knockout effects of the sg-B2M-1 and sg-B2M-2 reached more than 90%, which is significantly superior to that of the sg-B2M-3, indicating that both sg-B2M-1 and B2M-2 have good knockout effects.

**Table 4. Statistics of B2M knockout results using different gRNA sequences**

| gRNA | sg-B2M-1 | sg-B2M-2 | sg-B2M-3 |
|---|---|---|---|
| KO efficency (Day 5) | 95.0% | 90.0% | 67.0% |

Using the same sg-B2M-1 sequence, the publicly reported knockout rate can only reach 50-60% (Nature. 2017 Mar 2;543(7643):113-117, Fig. 3c showing that the B2M knockout rate is 55%). After optimizing through our method, the knockout rate of B2M is greatly improved to 95%.

### Example 7 Effects of Cas 9 enzyme concentrations on knockout efficiency

Sg-B2M-2 is selected as sgRNA. When the ratio of Cas 9 enzymes to sgRNAs is 1:4, different concentrations of the Cas 9 enzymes (0.125µM, 0.25µM, 0.5µM, 1.0 µM, 2.0 µM, 3.0 µM) are set to be detected for their effects on the B2M gene knockout. After incubating the RNP complex for 10 minutes at room temperature, the Maxcyte electrotransfection instrument (Maxcyte, Inc.) is used based on the electrotransfection conditions of the instrument to introduce the RNP complex into T cells. On the 5th day after transfection, the T cells are taken for B2M antibody (BD Biosciences) staining for flow cytometry to verify B2M knockout efficiency.

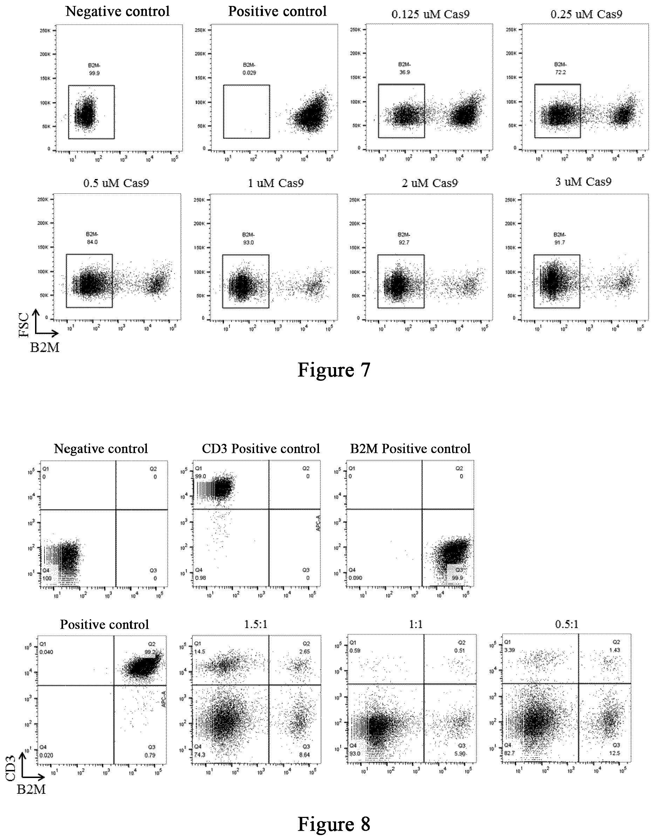

The results are shown in Figure 7 and Table 5. When the concentration of the Cas9 enzymes is greater than 0.2µM, the knockout efficiency can reach more than 70%. When at the concentration of 0.25µM, the knock-out efficiency is 72.2%; when the concentration of the Cas9 enzymes is no less than 1µM, the knock-out efficiency can reach more than 90%. For example, the knock-out efficiency is great at the concentration of 1µM-3µM, especially when at the concentration of 1µM- At 2µM, the knockout efficiency is around 93%.

**Table 5. Statistics of B2M knockout results using different concentrations of the Cas9 enzyme**

| | | | | | | |
|---|---|---|---|---|---|---|
| Cas9 enzyme concentration (RNP ratio 1: 4) | 0.125µM | 0.25µM | 0.5µM | 1µm | 2µM | 3µM |
| KO efficency (Day 5) | 36.9% | 72.2% | 84.0% | 93.0% | 92.7% | 91.7% |

### Example 8. Simultaneously and efficiently knockout of TRAC and B2M genes in T cells

### 1. The effect of gRNA components ratio

In the existing reports, the efficiency of TCR and B2M double knockout is only about 60% (refer to Figure 3b in Clin Cancer Res. 2017 May 1;23(9):2255-2266 and Fig3a in Oncotarget, 2017, Vol. 8.,(No.10), pp:17007-17011). Therefore, in this example, it is desired to further use the optimized method above to screen out the combination for efficient double knockout of the B2M and TCR.

In order to detect the effect of the ratio between sg-TRAC-1 and sg-B2M-2 on the double knockout of the TRAC and B2M genes, when the molar ratio of Cas9 enzymes to total gRNAs is 1:4, the ratio of the sg-B2M-2 to the sg-TRAC-1 is set to 1.5:1, 1:1 and 0.5:1 separately, and the effect on gene knockout is tested. After introducing an RNP complex into T cells using a Maxcyte electrotransfection instrument (Maxcyte, Inc.), CD3 antibody and B2M antibody (BD Biosciences) are stained for flow cytometry on the 5th day. The results of flow cytometry are shown in Figure 8 and Table 6. When the ratio of the sg-B2M-2 to the sg-TRAC-1 is 1:1, double knockout of the TRAC and B2M genes has the best effect.

**Table 6. Effects of different gRNA components on TRAC and B2M double knockout**

| gRNA ratio | 1.5:1 | 1:1 | 0.5:1 |
|---|---|---|---|
| KO efficiency (Day 5) | 74.3% | 93.0% | 82.7% |

### 2. Optimization of RNP concentration.

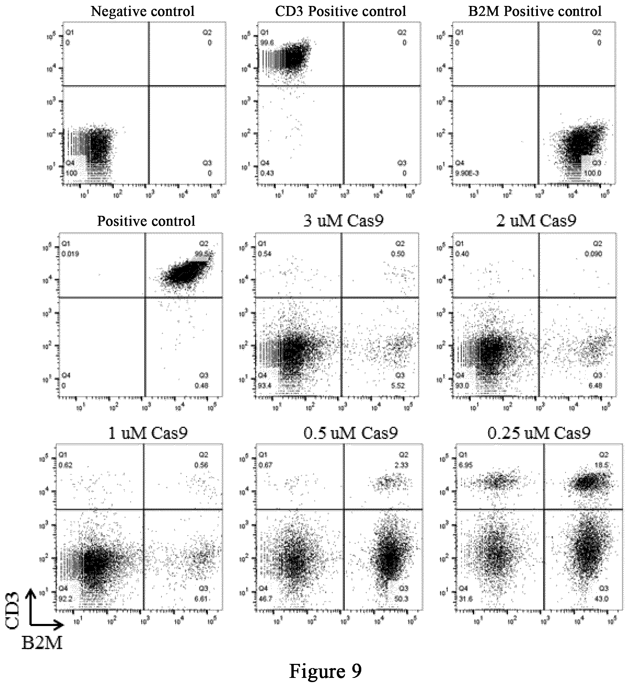

In order to explore the concentration of the RNP complex formed by the mixture composed of gRNAs targeting the TRAC and B2M genes and the Cas9 enzymes, when using the optimized molar ratio of Cas9 enzymes to gRNAs which is 1:4, different concentrations of the Cas 9 enzymes are set (0.25µM, 0.5µM, 1.0 µM, 2.0 µM, 3.0 µM) to be detected for their effects on gene knockout. After introducing the RNP complex into T cells using a Maxcyte electrotransfection instrument (Maxcyte, Inc.), CD3 antibody and B2M antibody (BD Biosciences) are stained for flow cytometry on the 5th day. The results of flow cytometry are shown in Figure 9 and Table 7. When the final concentration of Cas 9 enzyme is no less than 1µM, the effect of the TRAC and B2M double knockout could reach more than 90%, and when at the concentration of 3µM, it reachs to 93.4%.

**Table 7. Statistics of TRAC and B2M double knockout results using different concentrations of the Cas9 enzymes**

| | | | | | |
|---|---|---|---|---|---|
| Cas9 enzyme concentration (RNP ratio 1:4) | 0.25µM | 0.5µM | 1.0 µM | 2.0 µM | 3.0 µM |
| KO efficiency (Day 5) | 31.6% | 46.7% | 92.2% | 93.0% | 93.4% |

### Example 9. Verification of simultaneous knockout of TRAC and B2M genes in T cells on the molecular level

### 1. Verification of TRAC and B2M genes knockout with Tide method

The genomic DNA with one or both of TRAC and B2M genes knocked out are extracted from T cells, and the gene fragments comprising knockout sites are amplified by PCR. The PCR products are purified and recovered after gel electrophoresis, and then sequenced. The sequencing results of the TRAC and B2M genes in the PCR products of the control group are a single peak, while in the knockout group, the sequencing results of the TRAC and B2M genes will correspond to multiple sets of peaks, indicating that the TRAC and B2M genes are mutated.

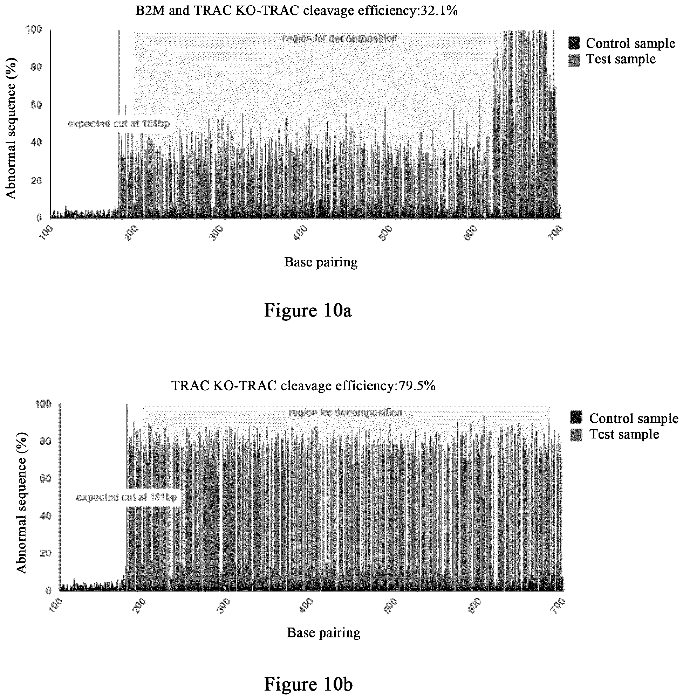

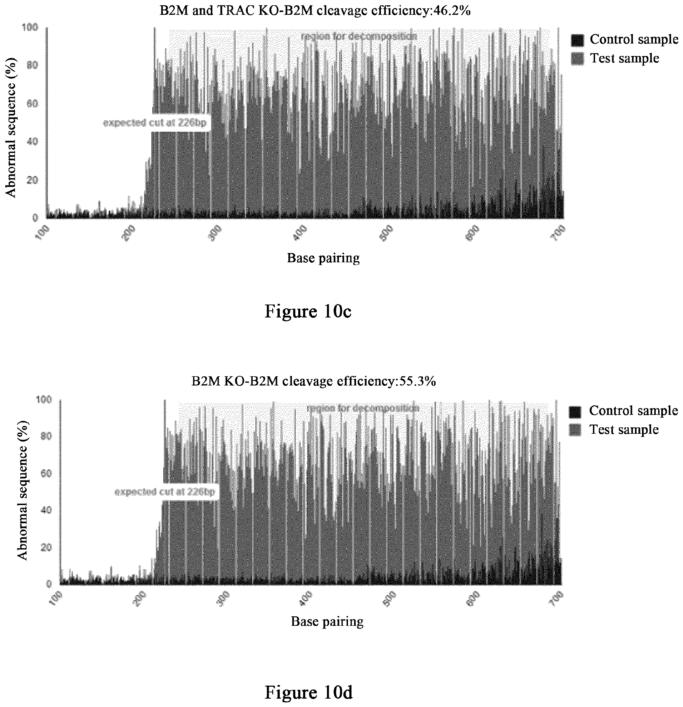

The sequencing results are submitted to https://tide.deskgen.com/ for analysis, and predicted mutation efficiencies are obtained. The results are shown in Figure 10, indicating that TCR and B2M are efficiently knocked out.

### 2. TRAC and B2M genes knockout verified by sequencing the clones.

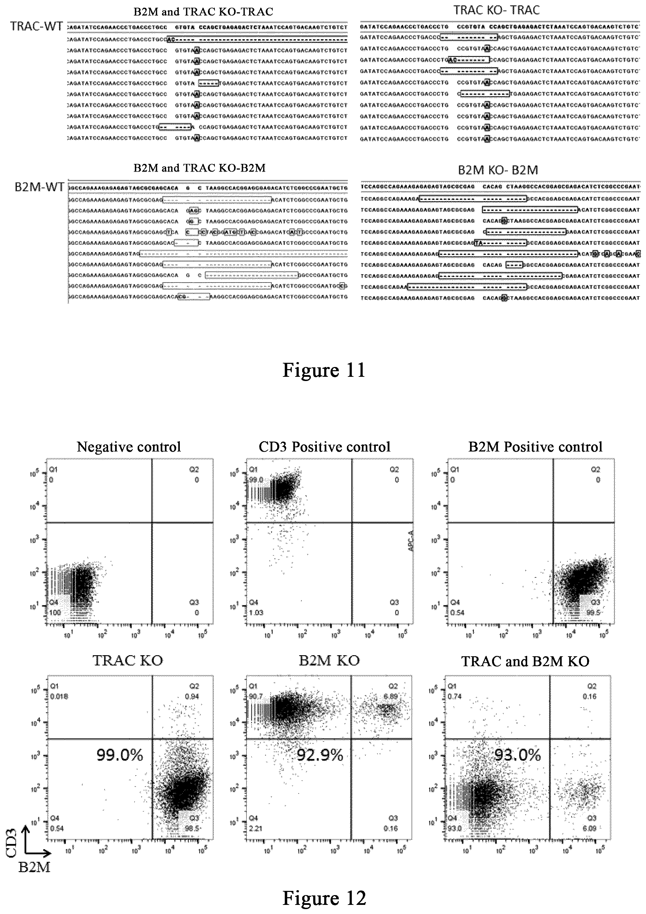

Genomic DNAs with one or both of TRAC and B2M genes knock-out are extracted from T cells separately, and gene fragments containing knock-out sites are amplified using PCR. The PCR products are purified and recovered after gel electrophoresis, and connected to T vectors and transformed. Monoclonal bacterial colonies are randomly picked for sequencing identification. As shown in Figure 11, the picked clones are compared by sequencing. Compared with the original sequences of the TRAC and B2M, all the sequences of the knockout groups shows base deletions or insertions, indicating that both TCR and B2M genes are mutated.

### Example 10: BCMA CAR-T cells with efficient knockout of TRAC and B2M genes

Furthermore, we used the prepared BCMA CAR-T cells to test the effect of TRAC and B2M double genes knockout.
1. Preparation of BCMA targeted-CAR-T cells. With reference to Chinese patent for invention 201810065525.1, a CAR vector comprising anti-BCMA chimeric antigen receptors, T cell costimulatory factor 41-BB, T cell activating factor CD3ζ is designed and constructed, and packed into a lentivirus. It is named PRRL-BCMA-BBZ(TM). After 48 hours of T cell activation and expansion, the cell density is adjucted to 2*10^6/mL. PRRL-BCMA-BBZ(TM) lentivirus are added at the ratio MOI=4, and the medium is changed after 24 hours. The target BCMA CAR-T cells are obtained.
2. Knockout of the TCR and B2M genes in the BCMA-targeted CAR-T cells. After 48 hours of *in vitro* expansion of CAR-T cells, the cell density is adjucted to 2*10^7/mL. Sg-TRAC-1, sg-B2M-2, and the mixture of sg-TRAC-1/sg-B2M-2 are separately incubated at room temperature for 10 minutes at a ratio of Cas 9 enzymes and gRNA of 1:4. 1*10^6 cells are mixed with RNPs (the final concentration of the Cas 9 enzyme is 3µM), and the RNP complex is introduced into the CAR-T cells using a maxcyte electrotransfection instrument. The cell viability is detected at 24 hours, 48 hours and 72 hours respectively (Table 8). CAR-T cells recovered well after electrotransfection. On the 5th day after electrotransfection, flow cytometry is used to detect the knockout of the TRAC and B2M genes. TRAC or B2M single gene knockout efficiency, and TRAC and B2M double gene knockout efficiency reached more than 90%, indicating that the TRAC and B2M double gene knockoutis efficiently achieved (see Figure 12).

**Table 8. Cell viability of CAR-T cells after electrotransfection**

| | **#1-TCR KO** | **#2-B2M KO** | **#3-B2M+TCR KO** | **#4-Blank control** |
|---|---|---|---|---|
| 24 h | 85% | 83% | 77% | 97% |
| 48 h | 85% | 87% | 91% | 93% |
| 72 h | 96% | 90% | 91% | 95% |

Sequences used herein are as follows:

| Names | SEQ ID NOs. | Sequences |
|---|---|---|
| TRAC-exon 1 sequence | 1 | ATATCCAGAACCCTGACCCTGCCGTGTACCAGCTGAGAGA CTCTAAATCCAGTGACAAGTCTGTCTGCCTATTCACCGATT TTGATTCTCAAACAAATGTGTCACAAAGTAAGGATTCTGA TGTGTATATCACAGACAAAACTGTGCTAGACATGAGGTCT ATGGACTTCAAGAGCAACAGTGCTGTGGCCTGGAGCAAC AAATCTGACTTTGCATGTGCAAACGCCTTCAACAACAGCA TTATTCCAGAAGACACCTTCTTCCCCAGCCCAGG |
| sg-TRAC-1 | 2 | AGAGTCTCTCAGCTGGTACA |
| sg-TRAC-2 | 3 | TCTCTCAGCTGGTACACGGC |
| sg-TRAC-3 | 4 | GAGAATCAAAATCGGTGAAT |
| sg-TRAC-4 | 5 | CTCTCAGCTGGTACACGGCA |
| sg-TRAC-1 correspondin g to TRAC | 6 | CCGTGTACCAGCTGAGAGACTCT |
| sg-TRAC-2 correspondin g to TRAC | 7 | CCTGCCGTGTACCAGCTGAGAGA |
| sg-TRAC-3 correspondi ng to TRAC | 8 | CCTATTCACCGATTTTGATTCTC |
| sg-TRAC-4 correspondi ng to TRAC | 9 | CCCTGCCGTGTACCAGCTGAGAG |
| B2M-exon 1 sequence | 10 | AATATAAGTGGAGGCGTCGCGCTGGCGGGCATTCCTGAAG CTGACAGCATTCGGGCCGAGATGTCTCGCTCCGTGGCCTT AGCTGTGCTCGCGCTACTCTCTCTTTCTGGCCTGGAGGCTA TCCAGC |
| sg-B2M-1 | 11 | GGCCACGGAGCGAGACATCT |
| sg-B2M-2 | 12 | GAGTAGCGCGAGCACAGCTA |
| sg-B2M-3 | 13 | GGCCGAGATGTCTCGCTCCG |
| sg-B2M-4 | 14 | AAGTGGAGGCGTCGCGCTGG |
| sg-B2M-1 correspondin g to B2M | 15 | CCGAGATGTCTCGCTCCGTGGCC |
| sg-B2M-2 correspondin g to B2M | 16 | CCTTAGCTGTGCTCGCGCTACTC |
| sg-B2M-3 correspondin g to B2M | 17 | GGCCGAGATGTCTCGCTCCGTGG |
| sg-B2M-4 correspondin g to B2M | 18 | AAGTGGAGGCGTCGCGCTGGCGG |
| scfv nucleic acid sequence of bcma antibody | 19 | GAGGTGCAATTGCTGGAGTCTGGGGGAGGCTTGGTACAGCCT GGGGGGTCCCTGAGACTCTCCTGTGCAGCCTCCGGATTCACC TTTGGCGGTAATGCCATGTCCTGGGTCCGCCAGGCTCCAGGG AAGGGGCTGGAGTGGGTCTCAGCAATTAGTGGTAATGGTGGT AGTACATTCTACGCAGACTCCGTGAAGGGCCGGTTCACCATC TCCAGAGACAATTCCAAGAACACGCTGTATCTGCAGATGAAC AGCCTGAGAGCCGAGGACACGGCCGTATATTACTGTGCGAAA GTTCGTCCATTCTGGGGTACTTTCGACTACTGGGGCCAAGGA ACCCTGGTCACCGTCTCGAGTGGTGGAGGCGGTTCAGGCGGA GGTGGTTCTGGCGGTGGCGGATCGGAAATCGTGTTAACGCAG TCTCCAGGCACCCTGTCTTTGTCTCCAGGGGAAAGAGCCACC CTCTCTTGCAGGGCCAGTCAGAGTGTTAGCAGCAGCTACTTA GCCTGGTACCAGCAGAAACCTGGCCAGGCTCCCAGGCTCCTC ATCTATGGAGCATCCAGCAGGGCCACTGGCATCCCAGACAGG TTCAGTGGCAGTGGATCCGGGACAGACTTCACTCTCACCATC AGCAGACTGGAGCCTGAAGATTTTGCAGTGTATTACTGTCAG CAGTACTTCAACCCACCAGAATACACGTTCGGCCAGGGGACC AAAGTGGAAATCAAACGT |
| sg-TRAC-1-F | 20 | TAATACGACTCACTATAGAGAGTCTCTCAGCTGGTACA |
| sg-TRAC-1-R | 21 | TTCTAGCTCTAAAACTGTACCAGCTGAGAGACTCT |
| sg-TRAC-2-F | 22 | TAATACGACTCACTATAGTCTCTCAGCTGGTACACGGC |
| sg-TRAC-2-R | 23 | TTCTAGCTCTAAAACGCCGTGTACCAGCTGAGAGA |
| sg-TRAC-3-F | 24 | TAATACGACTCACTATAGGAGAATCAAAATCGGTGAAT |
| sg-TRAC-3-R | 25 | TTCTAGCTCTAAAACATTCACCGATTTTGATTCTC |
| sg-B2M-1-F | 26 | TAATACGACTCACTATAGGGCCACGGAGCGAGACATCT |
| sg-B2M-1-R | 27 | TTCTAGCTCTAAAACAGATGTCTCGCTCCGTGGCC |
| sg-B2M-2-F | 28 | TAATACGACTCACTATAGGAGTAGCGCGAGCACAGCTA |
| sg-B2M-2-R | 29 | TTCTAGCTCTAAAACTAGCTGTGCTCGCGCTACTC |
| sg-B2M-3-F | 30 | TAATACGACTCACTATAGGGCCGAGATGTCTCGCTCCG |
| sg-B2M-3-R | 31 | TTCTAGCTCTAAAACCGGAGCGAGACATCTCGGCC |
| sg-TRAC-5 | 32 | GTCTCTCAGCTGGTACA |
| sg-TRAC-6 | 33 | AGTCTCTCAGCTGGTACA |
| sg-TRAC-5-F | 34 | TAATACGACTCACTATAGTTAGAGTCTCTCAGCTGGTACA |
| sg-TRAC-5-R | 35 | TTCTAGCTCTAAAACTGTACCAGCTGAGAGACTCTAA |
| sg-TRAC-6-F | 36 | TAATACGACTCACTATAGTTTAGAGTCTCTCAGCTGGTAC A |
| sg-TRAC-6-R | 37 | TTCTAGCTCTAAAACTGTACCAGCTGAGAGACTCTAAA |
| B2M editing target | 38 | TAGCTGTGCTCGCG |
| sg-TRAC-7 | 39 | TTAGAGTCTCTCAGCTGGTACA |
| sg-TRAC-8 | 40 | TTTAGAGTCTCTCAGCTGGTACA |
| sg-TRAC-7-F | 41 | TAATACGACTCACTATAGAGTCTCTCAGCTGGTACA |
| sg-TRAC-7-R | 42 | TTCTAGCTCTAAAACTGTACCAGCTGAGAGACT |
| sg-TRAC-8-F | 43 | TAATACGACTCACTATAGGTCTCTCAGCTGGTACA |
| sg-TRAC-8-R | 44 | TTCTAGCTCTAAAACTGTACCAGCTGAGAGAC |
| TRAC editing site | 45 | TGTACCAGCTGAGAG |

## Claims

1. A method for gene editing of a cell based on a CRISPR/Cas system, wherein a complex of a Cas enzyme and a gRNA is introduced into the cell for gene editing, wherein the Cas enzyme is a Cas9 enzyme, and enzyme activity of the Cas9 enzyme is 0.1 to 1 nmol, preferably 0.2 to 0.7 nmol, and more preferably 0.3 to 0.5 nmol;
more preferably, in the complex, the molar ratio of the Cas9 enzyme to the gRNA is 1:1 to 1:10, preferably 1:3 to 1:5, and more preferably 1:4.

2. The method of claim 1, wherein:
a first complex of the Cas9 enzyme and a first gRNA and a second complex of the Cas9 enzyme and a second gRNA are introduced into the cell for gene editing,
preferably, a third complex of the Cas9 enzyme, the first gRNA and the second gRNA are simultaneously introduced into the cell for gene editing,
wherein, in the first complex or the second complex or the third complex, the molar ratio of the Cas9 enzyme and the gRNA is 1:1∼1:10, preferably 1:3∼1:5, more preferably 1:4.

3. The method of claim 2, wherein the molar ratio of the first gRNA to the second gRNA is about 1:5 to 5:1, preferably 1:2 to 2:1; more preferably about 1:1.

4. The method according to any one of claims 1 to 3, wherein in the complex formed by the Cas9 enzyme and the gRNA or the first complex or the second complex or the third complex, the concentration of the Cas9 enzyme is bout 0.1µM∼3µM; preferably about 0.125µM∼3µM; more preferably about 0.2µM∼3µM; more preferably about 0.25µM∼3µM; more preferably about 0.5µM∼3µM; more preferably about 1µM to 3µM.

5. The method according to any one of claims 1 to 4, wherein the cell is a eukaryotic cell; preferably the eukaryotic cell is an immune effector cell; preferably the immune effector cell is a T cell.

6. The method of claim 5, wherein the cell is a T cell, and the CRISPR/Cas9 system is used for editing of a gene of the T cell; comprising:
using the CRISPR/Cas9 system to perform gene editing on genes of any one or two of an α chain and a β chain of TCR of the T cell; preferably to perform gene editing on TRAC; more preferably to perform gene editing on a constant region of the TRAC; more preferably to perform gene editing on the sequence shown in SEQ ID NO: 45 in the TRAC; more preferably to perform gene editing on the sequence shown in SEQ ID NO: 1 comprised in the TRAC, and/or
using the CRISPR/Cas9 system to perform gene editing on MHC gene of the T cell, preferably to perform gene editing on B2M gene, more preferably to perform gene editing on the sequence shown in SEQ ID NO: 38 in the B2M gene, and more preferably to perform gene editing on the sequence shown in SEQ ID NO: 10 comprised in the B2M gene.

7. The method according to any one of claims 1-6, wherein the gRNA is about 15-50 bp, preferably about 15-30 bp, more preferably about 17-21 bp; more preferably 20 bp .

8. The method of claim 7, wherein the gRNA adopted for editing the TRAC comprises the sequence shown in SEQ ID NO: 2, 3, 4, 5, 32, 33, 39 or 40; preferably, the gRNA adopted comprises the sequence shown in SEQ ID NO: 2, 32 or 33.

9. The method of claim 7, wherein the gRNA adopted for the B2M gene editing comprises the sequence shown in SEQ ID NO: 11, 12, 13, or 14; preferably, the adopted gRNA comprises the sequence shown in SEQ ID NO: 12.

10. The method according to any one of claims 5-9, wherein the T cell also expresses a chimeric receptor, an exogenous cytokine, an inhibitory/activating receptor or ligand, a costimulating factor; preferably, the T cell further expresses a chimeric antigen receptor.

11. A method for constructing an universal T cell, comprising:
gene editing of TCR gene and MHC gene of a T cell with a gene editing technology, wherein
for gene editing of the TCR gene of the T cell, it is preferable to perform gene editing on genes of any one or two of an α chain and a β chain of TCR of the T cell, preferably to perform gene editing on the TRAC gene, more preferably to perform gene editing on a constant region of the TRAC, more preferably to perform gene editing on the sequence shown in SEQ ID NO:45 in the TRAC, and more preferably to perform gene editing on the sequence shown in SEQ ID NO: 1 comprised in the TRAC; and
for gene editing of the MHC gene of the T cell, it is preferably to perform gene editing on a B2M gene of the T cell, more preferably to perform gene editing on the sequence shown in SEQ ID NO: 38 in the B2M gene, and more preferably to perform gene editing on the sequence shown in SEQ ID NO: 10 comprised in the B2M gene.

12. The method of claim 11, wherein the gene editing technology is a CRISPR/Cas9 gene editing technology.

13. The method of claim 11 or 12, wherein,
a gRNA comprising the sequence shown in SEQ ID NO: 2, 3, 4, 5, 32, 33, 39, or 40 is introduced into the T cell to achieve gene editing of the TRAC gene of the T cell, and preferably the gRNA comprising the sequence shown in SEQ ID NO: 2, 32 or 33 is introduced into the T cell to achieve gene editing of the TRAC gene of the T cell.

14. The method of claim 12 or 13, wherein:
a gRNA comprising the sequence shown in SEQ ID NO: 11, 12, 13, or 14 is introduced into the T cell to achieve gene editing of the MHC gene of the T cell, and preferably the gRNA comprising the sequence shown in SEQ ID NO: 12 is introduced into the T cell to achieve gene editing of the MHC gene of the T cell.

15. The method of any one of claims 11-14, wherein:
gene editing of TCR gene of the T cell is conducted using the gene editing technology by introducing a first complex of the Cas9 enzyme and gRNA into the cell for gene editing,
gene editing of MHC gene of the T cell is conducted using the gene editing technology by introducing a second complex of the Cas9 enzyme and gRNA into the cell for gene editing,
preferably, the first complex and the second complex are simultaneously introduced into the cell in the form of a third complex for gene editing.

16. The method of claim 15, wherein:
the molar ratio of the Cas9 enzyme and the gRNA in the first complex or the second complex is 1:1 to 1:10, preferably 1:3 to 1:5, and more preferably the molar ratio of the Cas9 enzyme to the gRNA is 1:4.

17. The method of claim 15, wherein:
in the first complex or the second complex or the third complex, the concentration of the Cas9 enzyme is about 0.1µM to 3µM; preferably about 0.125µM to 3µM; more preferably about 0.2µM to 3µM; more preferably about 0.25µM to 3µM; more preferably about 0.5µM to 3µM, more preferably about 1µM to 3µM, more preferably about 0.125µM to 0.5µM, more preferably about 0.25µM to 0.5µM.

18. The method of any one of claims 11-17, wherein:
the molar ratio of the gRNA for gene editing of the TCR gene and the gRNA for gene editing of the MHC gene is about 1:5 to 5:1, preferably 1:2 to 2:1; more preferably, about 1:1.

19. The method according to any one of claims 11-18, wherein the T cell further expresses a chimeric antigen receptor, preferably the T cell further expresses a chimeric receptor that recognizes a tumor antigen or a pathogen antigen, wherein the chimeric receptor has an extracellular antigen binding domain, a transmembrane domain, and an intracellular domain, and the extracellular antigen binding domain specifically recognizes the target antigen;
preferably, the target antigen is a tumor antigen selected from the group consisting of: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3(CD276), B7H6; KIT (CD117); interleukin 13 receptor subunit α (IL -13Rα); interleukin 11 receptor α (IL-11Rα); prostate stem cell antigen (PSCA); prostate specific membrane antigen (PSMA); carcinoembryonic antigen (CEA); NY-ESO-1; HIV-1 Gag; MART -1; gp100; tyrosinase; mesothelin; EpCAM; protease serine 21 (PRSS21); vascular endothelial growth factor receptor; Lewis (Y) antigen; CD24; platelet-derived growth factor receptor β (PDGFR-β); stage-specific embryonic antigen-4 (SSEA-4); cell surface-associated mucin 1 (MUC1), MUC6; epidermal growth factor 20 receptor family and its mutants (EGFR, EGFR2, ERBB3, ERBB4, EGFRvIII); nerve cell adhesion molecule (NCAM); carbonic anhydrase IX (CAIX); LMP2; ephrin A receptor 2 (EphA2); fucosyl GM1; sialyl Lewis adhesion molecule (sLe); O-acetyl GD2 ganglioside (OAcGD2); ganglioside GM3; TGS5; high molecular weight melanoma-associated antigen (HMWMAA); folate receptor; tumor vascular endothelial marker 251 (TEM1/CD248); tumor vascular endothelial marker 7 related (TEM7R); Claudin6, Claudin18.2 (CLD18A2), Claudin18.1; ASGPR1; CDH16; 5T4; 8H9; αvβ6 integrin; B cell maturation antigen (BCMA); CA9; κ light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis region; G protein-coupled receptor, family C, group 5, member D (GPRC5D); X chromosome open reading frame 61 (CXORF61); CD97; CD179a; anaplastic lymphoma kinase (ALK); polysialic acid; placenta specific 1 (PLAC1); hexose part of globoH glycoceramide (GloboH); breast differentiation antigen (NY-BR-1); uroplakin 2 (UPK2); hepatitis A virus cell receptor 1 (HAVCR1); adrenaline receptor 5 β3 (ADRB3); pannexin 3 (PANX3); G protein coupled receptor 20 (GPR20); lymphocyte antigen 6 complex locus K9 (LY6K); olfactory receptor 51E2 (OR51E2); TCRγ alternating reading frame protein (TARP); Wilms tumor protein (WT1); ETS translocation variant gene 6 (ETV6-AML); sperm protein 17 (SPA17); X antigen family member 1A (XAGE1); angiopoietin binding cell-surface receptor 2 (Tie2); melanoma cancer testis antigen-1 (MAD-CT-1); melanoma cancer testis antigen-2 (MAD-CT-2); Fos-related antigen 1; p53 mutant 10; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin B1; V-myc avian myelocytomatosis virus oncogene neuroblastoma-derived homolog (MYCN); Ras homolog family member C(RhoC); Cytochrome P450 1B1 (CYP1B1); CCCTC binding factor (zinc finger protein)-like (BORIS); Squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte associated immunoglobulin like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12, member A (CLEC12A); bone marrow stromal cell antigen 2 (BST2); EGF-like module-containing mucin-like hormone receptor-like 2 (EMR2); lymphocyte antigen 75 (LY75); glypican-3 (GPC3); Fc receptor-like 5 (FCRL5); immunoglobulin lambda-like polypeptide 1 (IGLL1);
preferably, the target antigen is a pathogen antigen, and the pathogen antigen is selected from the group consisting of: virus, bacteria, fungus, protozoa, or parasite antigen; in one embodiment, the virus antigen is selected from the group consisting of cytomegalovirus antigen, Epstein-Barr virus antigen, human immunodeficiency virus antigen or influenza virus antigen.

20. The method of claim 19, wherein the chimeric receptor is a chimeric antigen receptor (CAR).

21. The method of claim 20, wherein the chimeric antigen receptor comprises:
(i) an antibody or a fragment thereof that specifically binds to a tumor antigen, a transmembrane region of CD28 or CD8, a costimulatory signal domain of CD28, and CD3ζ; or
(ii) an antibody or a fragment thereof that specifically binds to a tumor antigen, a transmembrane region of CD28 or CD8, a costimulatory signal domain of CD137, and CD3ζ; or
(iii) an antibody or a fragment thereof that specifically binds to a tumor antigen, a transmembrane region of CD28 or CD8, a costimulatory signal domain of CD28, a costimulatory signal domain of CD137, and CD3ζ.

22. The method of claim 20, wherein the antibody of the chimeric antigen receptor that specifically binds to a tumor antigen is a full-length antibody, scFv, Fab, (Fab'), or a single domain antibody.

23. Use of a T cell prepared according to the method of any one of claims 11-22 for preparing a chimeric receptor-expressing T cell, the chimeric receptor has an extracellular antigen binding domain, a transmembrane domain, and an intracellular domain, wherein, the extracellular antigen binding domain specifically recognizes a target antigen.

24. A universal T cell constructed according to the method of any one of claims 11-22.

25. A universal T cell, wherein a TRAC and/or a B2M gene are silenced.

26. The T cell of claim 25, wherein the TRAC gene is silenced by gene editing a sequence comprising the sequence shown in SEQ ID NO:1, and more preferably, the TRAC gene is silenced by gene editing the sequence shown in SEQ ID NO: 45 in the sequence comprising the sequence shown in SEQ ID NO: 1;
the B2M gene is silenced by gene editing of a sequence comprising the sequence shown in SEQ ID NO: 10, and more preferably, the B2M gene is silenced by gene editing the sequence shown in SEQ ID NO: 38 in a sequence comprising the sequence shown in SEQ ID NO: 10;

27. The T cell of claim 25, wherein the TRAC gene is silenced by gene editing of the TRAC gene using a gRNA of the sequence shown in SEQ ID NO: 2, 32 or 33,
B2M gene is silenced by gene editing of the B2M gene using a gRNA of the sequence shown in SEQ ID NO: 12.

28. The T cell according to any one of claims 24-27, wherein the T cell further expresses a chimeric antigen receptor, preferably the T cell further expresses a chimeric receptor that recognizes a tumor antigen or a pathogen antigen, the chimeric receptor has an extracellular antigen binding domain, a transmembrane domain, and an intracellular domain, and the extracellular antigen binding domain specifically recognizes a target antigen.

29. A gRNA construct, comprising a nucleotide sequence selected from the group consisting of SEQ ID NO: 2, 3, 4, 5, 32, 33, 39, 40, 11, 12, 13, or 14.

30. The gRNA construct of claim 20, comprising:
a nucleotide sequence selected from the group consisting of SEQ ID NO: 2, 3, 4, 5, 32, 33, 39, or 40, and
a nucleotide sequence selected from the group consisting of SEQ ID NO: 11, 12, 13, or 14.


SEQUENCE LISTING

<110>  CAFA THERAPEUTICS LIMITED

<120>  METHOD FOR GENE EDITING OF CELL ON THE BASIS OF CRISPR/CAS SYSTEM

<130>  BET 21M1110

<140>  EP19863140.0
<111>  2019-09-23

<150>  CN201811117875 .4
<151>  2018-09-21

<150>  CN201811140870.3
<151>  2018-09-28

<150>  CN201910809475.8
<151>  2019-08-29

<150>  CN201910809598.1
<151>  2019-08-29

<160>  45

<170>  PatentIn version 3.5

<210>  1
<211>  274
<212>  DNA
<213>  Artificial Sequence

<220>
<223>  TRAC-exon 1 sequence

<400>  1
atatccagaa ccctgaccct gccgtgtacc agctgagaga ctctaaatcc agtgacaagt      60

ctgtctgcct attcaccgat tttgattctc aaacaaatgt gtcacaaagt aaggattctg     120

atgtgtatat cacagacaaa actgtgctag acatgaggtc tatggacttc aagagcaaca     180

gtgctgtggc ctggagcaac aaatctgact ttgcatgtgc aaacgccttc aacaacagca     240

ttattccaga agacaccttc ttccccagcc cagg                                 274


<210>  2
<211>  20
<212>  DNA
<213>  Artificial Sequence

<220>
<223>  sg-TRAC-1

<400>  2
agagtctctc agctggtaca                                                  20


<210>  3
<211>  20
<212>  DNA
<213>  Artificial Sequence

<220>
<223>  sg-TRAC-2

<400>  3
tctctcagct ggtacacggc                                                    20


<210>  4
<211>  20
<212>  DNA
<213>  Artificial Sequence

<220>
<223>  sg-TRAC-3

<400>  4
gagaatcaaa atcggtgaat                                                    20


<210>  5
<211>  20
<212>  DNA
<213>  Artificial Sequence

<220>
<223>  sg-TRAC-4

<400>  5
ctctcagctg gtacacggca                                                    20


<210>  6
<211>  23
<212>  DNA
<213>  Artificial Sequence

<220>
<223>  TRAC corresponding to sg-TRAC-1

<400>  6
ccgtgtacca gctgagagac tct                                                23


<210>  7
<211>  23
<212>  DNA
<213>  Artificial Sequence

<220>
<223>  TRAC corresponding to sg-TRAC-2

<400>  7
cctgccgtgt accagctgag aga                                                23


<210>  8
<211>  23
<212>  DNA
<213>  Artificial Sequence

<220>
<223>  TRAC corresponding to sg-TRAC-3

<400>  8
cctattcacc gattttgatt ctc                                              23


<210>  9
<211>  23
<212>  DNA
<213>  Artificial Sequence

<220>
<223>  TRAC corresponding to sg-TRAC-4

<400>  9
ccctgccgtg taccagctga gag                                              23


<210>  10
<211>  127
<212>  DNA
<213>  Artificial Sequence

<220>
<223>  B2M-exon 1 sequence

<400>  10
aatataagtg gaggcgtcgc gctggcgggc attcctgaag ctgacagcat tcgggccgag      60

atgtctcgct ccgtggcctt agctgtgctc gcgctactct ctctttctgg cctggaggct     120

atccagc                                                               127


<210>  11
<211>  20
<212>  DNA
<213>  Artificial Sequence

<220>
<223>  sg-B2M-1

<400>  11
ggccacggag cgagacatct                                                  20


<210>  12
<211>  20
<212>  DNA
<213>  Artificial Sequence

<220>
<223>  sg-B2M-2

<400>  12
gagtagcgcg agcacagcta                                                  20


<210>  13
<211>  20
<212>  DNA
<213>  Artificial Sequence

<220>

<223>  sg-B2M-3

<400>  13
ggccgagatg tctcgctccg                                                    20


<210>  14
<211>  20
<212>  DNA
<213>  Artificial Sequence

<220>
<223>  sg-B2M-4

<400>  14
aagtggaggc gtcgcgctgg                                                    20


<210>  15
<211>  23
<212>  DNA
<213>  Artificial Sequence

<220>
<223>  B2M corresponding to sg-B2M-1

<400>  15
ccgagatgtc tcgctccgtg gcc                                                23


<210>  16
<211>  23
<212>  DNA
<213>  Artificial Sequence

<220>
<223>  B2M corresponding to sg-B2M-2

<400>  16
ccttagctgt gctcgcgcta ctc                                                23


<210>  17
<211>  23
<212>  DNA
<213>  Artificial Sequence

<220>
<223>  B2M corresponding to sg-B2M-3

<400>  17
ggccgagatg tctcgctccg tgg                                                23


<210>  18
<211>  23
<212>  DNA
<213>  Artificial Sequence

<220>
<223>  B2M corresponding to sg-B2M-4

<400>  18

aagtggaggc gtcgcgctgg cgg                                              23

<210>  19
<211>  732
<212>  DNA
<213>  Artificial Sequence

<220>
<223>  nucleotide sequence for scfv of bvma antibody

<400>  19
gaggtgcaat tgctggagtc tgggggaggc ttggtacagc ctggggggtc cctgagactc      60

tcctgtgcag cctccggatt cacctttggc ggtaatgcca tgtcctgggt ccgccaggct     120

ccagggaagg ggctggagtg ggtctcagca attagtggta atggtggtag tacattctac     180

gcagactccg tgaagggccg gttcaccatc tccagagaca attccaagaa cacgctgtat     240

ctgcagatga acagcctgag agccgaggac acggccgtat attactgtgc gaaagttcgt     300

ccattctggg gtactttcga ctactggggc caaggaaccc tggtcaccgt ctcgagtggt     360

ggaggcggtt caggcggagg tggttctggc ggtggcggat cggaaatcgt gttaacgcag     420

tctccaggca ccctgtcttt gtctccaggg gaaagagcca ccctctcttg cagggccagt     480

cagagtgtta gcagcagcta cttagcctgg taccagcaga aacctggcca ggctcccagg     540

ctcctcatct atggagcatc cagcagggcc actggcatcc cagacaggtt cagtggcagt     600

ggatccggga cagacttcac tctcaccatc agcagactgg agcctgaaga ttttgcagtg     660

tattactgtc agcagtactt caacccacca gaatacacgt tcggccaggg gaccaaagtg     720

gaaatcaaac gt                                                         732

<210>  20
<211>  38
<212>  DNA
<213>  Artificial Sequence

<220>
<223>  sg-TRAC-1-F

<400>  20
taatacgact cactatagag agtctctcag ctggtaca                             38

<210>  21
<211>  35
<212>  DNA
<213>  Artificial Sequence

<220>
<223>  sg-TRAC-1-R

<400>  21
ttctagctct aaaactgtac cagctgagag actct                                35

<210>  22
<211>  38
<212>  DNA
<213>  Artificial Sequence

<220>
<223>  sg-TRAC-2-F

<400>  22
taatacgact cactatagtc tctcagctgg tacacggc                              38


<210>  23
<211>  35
<212>  DNA
<213>  Artificial Sequence

<220>
<223>  sg-TRAC-2-R

<400>  23
ttctagctct aaaacgccgt gtaccagctg agaga                                 35


<210>  24
<211>  38
<212>  DNA
<213>  Artificial Sequence

<220>
<223>  sg-TRAC-3-F

<400>  24
taatacgact cactatagga gaatcaaaat cggtgaat                              38


<210>  25
<211>  35
<212>  DNA
<213>  Artificial Sequence

<220>
<223>  sg-TRAC-3-R

<400>  25
ttctagctct aaaacattca ccgattttga ttctc                                 35


<210>  26
<211>  38
<212>  DNA
<213>  Artificial Sequence

<220>
<223>  sg-B2M-1-F

<400>  26
taatacgact cactataggg ccacggagcg agacatct                              38


<210>  27
<211>  35
<212>  DNA

<213>  Artificial Sequence

<220>
<223>  sg-B2M-1-R

<400>  27
ttctagctct aaaacagatg tctcgctccg tggcc                                35


<210>  28
<211>  38
<212>  DNA
<213>  Artificial Sequence

<220>
<223>  sg-B2M-2-F

<400>  28
taatacgact cactatagga gtagcgcgag cacagcta                             38


<210>  29
<211>  35
<212>  DNA
<213>  Artificial Sequence

<220>
<223>  sg-B2M-2-R

<400>  29
ttctagctct aaaactagct gtgctcgcgc tactc                                35


<210>  30
<211>  38
<212>  DNA
<213>  Artificial Sequence

<220>
<223>  sg-B2M-3-F

<400>  30
taatacgact cactataggg ccgagatgtc tcgctccg                             38


<210>  31
<211>  35
<212>  DNA
<213>  Artificial Sequence

<220>
<223>  sg-B2M-3-R

<400>  31
ttctagctct aaaaccggag cgagacatct cggcc                                35


<210>  32
<211>  17
<212>  DNA
<213>  Artificial Sequence

<220>

<223>  sg-TRAC-5

<400>  32
gtctctcagc tggtaca                                                    17


<210>  33
<211>  18
<212>  DNA
<213>  Artificial Sequence

<220>
<223>  sg-TRAC-6

<400>  33
agtctctcag ctggtaca                                                   18


<210>  34
<211>  40
<212>  DNA
<213>  Artificial Sequence

<220>
<223>  sg-TRAC-5-F

<400>  34
taatacgact cactatagtt agagtctctc agctggtaca                           40


<210>  35
<211>  37
<212>  DNA
<213>  Artificial Sequence

<220>
<223>  sg-TRAC-5-R

<400>  35
ttctagctct aaaactgtac cagctgagag actctaa                              37


<210>  36
<211>  41
<212>  DNA
<213>  Artificial Sequence

<220>
<223>  sg-TRAC-6-F

<400>  36
taatacgact cactatagtt tagagtctct cagctggtac a                         41


<210>  37
<211>  38
<212>  DNA
<213>  Artificial Sequence

<220>
<223>  sg-TRAC-6-R

<400>  37

ttctagctct aaaactgtac cagctgagag actctaaa                                38

<210>  38
<211>  14
<212>  DNA
<213>  Artificial Sequence

<220>
<223>  target on B2M

<400>  38
tagctgtgct cgcg                                                          14

<210>  39
<211>  22
<212>  DNA
<213>  Artificial Sequence

<220>
<223>  sg-TRAC-7

<400>  39
ttagagtctc tcagctggta ca                                                 22

<210>  40
<211>  23
<212>  DNA
<213>  Artificial Sequence

<220>
<223>  sg-TRAC-8

<400>  40
tttagagtct ctcagctggt aca                                                23

<210>  41
<211>  36
<212>  DNA
<213>  Artificial Sequence

<220>
<223>  sg-TRAC-7-F

<400>  41
taatacgact cactatagag tctctcagct ggtaca                                  36

<210>  42
<211>  33
<212>  DNA
<213>  Artificial Sequence

<220>
<223>  sg-TRAC-7-R

<400>  42
ttctagctct aaaactgtac cagctgagag act                                     33

<210>  43
<211>  35
<212>  DNA
<213>  Artificial Sequence

<220>
<223>  sg-TRAC-8-F

<400>  43
taatacgact cactataggt ctctcagctg gtaca                                35


<210>  44
<211>  32
<212>  DNA
<213>  Artificial Sequence

<220>
<223>  sg-TRAC-8-R

<400>  44
ttctagctct aaaactgtac cagctgagag ac                                   32


<210>  45
<211>  15
<212>  DNA
<213>  Artificial Sequence

<220>
<223>  site for editing on TRAC

<400>  45
tgtaccagct gagag                                                      15